# EUROPÄISCHE PATENTSCHRIFT

(11) **EP 0 270 982 B1**
(45) Veröffentlichungstag und Bekanntmachung des Hinweises auf die Patenterteilung: **13.04.1994**
(21) Anmeldenummer: 87117748.1
(22) Anmeldetag: 01.12.1987
(51) Int. Cl.: C07D 401/12

(54) **Derivate bicyclischer Aminocarbonsäuren, Verfahren und Zwischenprodukte zu deren Herstellung sowie deren Verwendung**
Bicyclic derivatives of aminoacids, process and intermediates for their preparation, and their use
Dérivés bicycliques d'acides-aminés, procédé et intermédiaires de préparation et utilisation

(30) Priorität: 04.12.1986 DE 3641451
(43) Veröffentlichungstag der Anmeldung: 15.06.1988
(73) Patentinhaber: HOECHST AKTIENGESELLSCHAFT, 65926 Frankfurt am Main (DE)
(72) Erfinder: Barton, Derek, College Station, Texas 77840 (US); Potier, Pierre, F-75007 Paris (FR); Hervé,Yolande, F-92800 Puteaux (FR); Thierry, Josiane, F-91190 Gif-sur-Yvette (FR)

(56) Entgegenhaltungen:
- EP-A- 0 084 164
- TETRAHEDRON LETTERS, Band 28, Nr. 13, 1987, Seiten 1413-1416, Pergamon Press LTd., Oxford, GB; D.H.R. BARTON et al.: "Synthesis of 2S, 3aS, 7aS- and of 2S, 3aR, 7aR-perhydroindole-2-carboxylic acid derivatives from L-aspartic acid"
- TETRAHEDRON LETTERS, Band 42, Nr. 18, 1986, Seiten 4983-4990, Pergamon Press Ltd., Oxford, GB; D.H.R. BARTON et al.: "Concise syntheses of L-selenomethionine and L-selenocystine using radical chain reactions"

## Beschreibung

Acylderivate der Octahydroindol-2-carbonsäure, der Octahydrocyclopenta [b]pyrrol-2-carbonsäure bzw. der Decahydrocyclohepta [b]pyrrol-2-carbonsäure sind beispielsweise bekannt aus EP-A-79022, EP-A-50800, EP-A-84164, EP-A-111873, EP-A-37231, US-Patent 4 350 704 oder US-Patent 4 587 258. Viele dieser Verbindungen zeigen eine bermerkenswerte biologisch Aktivität. Sie hemmen beispielsweise hochwirksam das Angiotensin-Converting-Enzyme oder zeichnen sich durch eine nootrope Wirkung aus.

Verbindungen der Formel IV 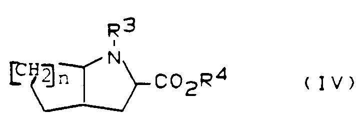
worin R³ Wasserstoff oder einen Acylrest und R⁴ Wasserstoff, eine Estergruppe oder eine andere Carboxylschutzgruppe bedeuten, spielen eine Schlüsselrolle bei der Synthese der eingangs genannten Acylderivate.

Oft ist es vorteilhaft, wenn das Kohlenstoffatom C-2 in Position 2 des bicyclischen Ringsystems dieser Wirkstoffe eine bestimmte absolute Konfiguration, vorzugsweise die S-Konfiguration aufweist. Man geht daher bei ihrer Synthese bevorzugt von Zwischenprodukten der Formel IV aus, welche diese gewünschte Konfiguration am C-2 bereits aufweisen.

Bei den bereits bekannten Herstellungsverfahren für Verbindungen der Formel IV war eine Racematspaltung unverzichtbar, wollte man zu Verbindungen mit einer definierten Konfiguration am C-2 gelangen.

Es wurde nun gefunden, daß sich Thiohydroxamsäurederivate insbesondere N-Hydroxy-2-thiopyridon-Derivate entsprechend substituierter und konfigurierter Aspariginsäure durch Cyclisierung und anschließende Abspaltung eines 2-Thioxo-2H[1] pyridyl-Restes in optisch einheitliche Verbindungen der Formel IV mit der gewünschten Konfiguration am C-2 überführen lassen, ohne daß auf irgend einer Stufe dieses neuen Verfahrens eine Racematspaltung notwendig wäre. Verbindungen der Formel I 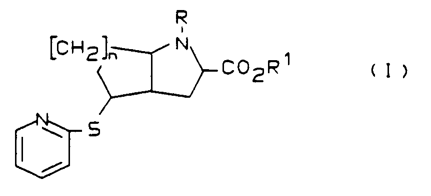
sind wichtige Zwischenprodukte dieses Verfahrens.

Die Erfindung betrifft daher Verbindungen der Formel I,
in welcher
n = 1, 2 oder 3
R (C₁-C₁₂)-Acyl und
R¹ (C₁-C₆)-Alkyl, (C₃-C₇)-Cycloalkyl, (C₇-C₁₁)-Aralkyl oder eine andere Carboxylschutzgruppe bedeuten, wobei die Wasserstoffatome an den Brückenkopf-Kohlenstoffatomen 3a und (5+n) a vorzugsweise cis-konfiguriert sind.

Das Kohlenstoffatomen in Position 2 des bicyclischen Ringsystems kann sowohl die R- als auch die S-Konfiguration aufweisen; bevorzugt ist die S-Konfiguration.

R ist vorzugsweise (C₁-C₆)-Alkanoyl, (C₆-C₁₀)-Aryl-(C₁-C₄)-alkanoyl, (C₆-C₁₀)-Aroyl, (C₁-C₆)-Alkoxycarbonyl oder (C₇-C₁₁)-Aralkyloxycarbonyl, insbesondere aber (C₁-C₄)-Alkanoyl, wie Acetyl oder Propionyl, oder Benzoyl oder substituiertes Benzoyl.

Des weiteren kann R, falls von den vorstehenden Definitionen nicht schon umfaßt, für eine in der Peptidchemie übliche Aminschutzgruppe vom Urethantyp stehen (vgl. z.B. Hubbuch, Kontakte Merck 3/79, 14-22). Schutzgruppen vom Urethantyp sind beispielsweise Pyoc, Fmoc, Tcboc, Z, Boc, Ddz, Bpoc, Adoc, Msc, Moc, Z(NO₂), Z(Halₙ), Dobz, Iboc, Adpoc, Mboc und 1,4-Dimethyl-pyridyl-oxycarbonyl.

R¹ ist vorzugsweise (C₁-C₄)-Alkyl, wie z.B. Methyl, Ethyl oder tert.-Butyl, oder (C₇-C₁₁)-Aralkyl, wie beispielsweise Benzyl.

Weiterhin kann R¹, falls von den vorstehenden Definitionen nicht schon umfaßt, für eine in der Peptidchemie übliche Carboxylschutzgruppe stehen (vgl. z.B. oben genannten Artikel von Hubbuch), Carboxylschutzgruppen beispielsweise die schon erwähnte Alkylreste oder Benzyl. Weiter sind modifizierte Benzylreste, wie p-Nitrobenzyl, p-Methoxybenzyl, p-Brombenzyl, p-Chlorobenzyl und Reste wie 4-Picolyl oder Benzoylmethyl geeignet. Unter Alkyl wird im verstehenden und im folgenden geradkettiges oder verzweigtes Alkyl verstanden. Ensprechendes gilt für davon abgeleitete Reste wie z.B. Alkanoyl und Aralkyl. Niederalkyl weist vorzugsweise bis zu 6 C-Atome auf. (C₈-C₁₀)-Aryl ist beispielsweise Phenyl oder Naphthyl; bevorzugt ist Phenyl. Entsprechendes gilt für davon abgeleitete Reste wie z.B. Aroyl und Aralkyl.

Die Erfindung betrifft weiterhin eine Verfahren zur Herstellung von Verbindungen der Formel I, das dadurch gekennzeichnet ist, daß man eine Verbindung der Formel II 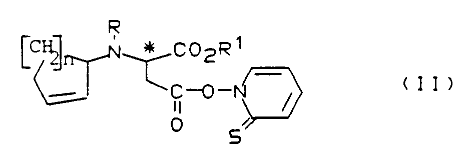
in welcher n, R und R¹ wie oben definiert sind, radikalisch decarboxyliert.

Die radikalische Decarboxylierung kann z.B. durch Erwärmen auf 40-190° C, vorzugsweise 80-130°C in einem geeigneten Lösungsmittel oder auch ohne Lösungsmittel gegebenenfalls in Gegenwart eines Radikalinitiators durchgeführt werden. Als Lösungsmittel kommen dabei insbesondere aprotische Lösungsmittel wie Benzol, Toluol oder Xylol in Frage. Geeignete Initiatoren sind beispielsweise organische Peroxide, wie tert.-Butylperoxid, substituierte Azo-acetonitrile.

Des weiteren kann die radikalische Decarboxylierung photolytisch oder radiolytisch in einem geeigneten dipolar aprotischen Lösungsmittel zwischen -20°C und dem Siedepunkt des Reaktionsgemisches vorzugsweise zwischen 10 und 50° C durchgeführt werden. Die photolytische Decarboxylierung ist bevorzugt. Geeignete dipolar aprotische Lösungsmittel sind beispielsweise Ether, wie Diethylether, Tetrahydrofuran und Dioxan.

Man stellt die Verbindungen der Formel II ausgehend von Cycloalkenylbromiden der Formel V, 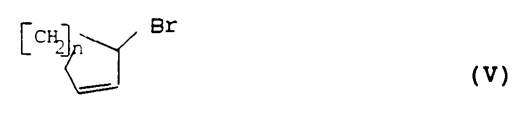
worin n 1, 2 oder 3 ist, her. Diese werden mit Aspariginsäurederivaten der Formel VI, 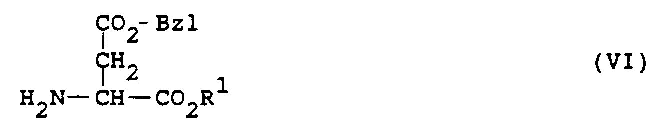
in der R¹ wie oben definiert ist, vorzugsweise (C₁-C₆)-Alkyl wie tert. Butyl bedeuten und die vorzugsweise L-konfiguriert sind, in Gegenwart einer Base wie K₂CO₃ in einem dipolar aprotischen Lösungsmittel wie Acetonitril zwischen 0° C und dem Siedepunkt des Reaktionsgemisches, vorzugsweise bei Raumtemperatur zu Verbindungen der Formel VII, worin n und R¹ wie oben definiert sind, umgesetzt. 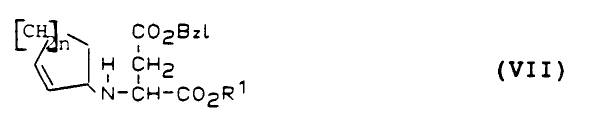
Diese werden zu Verbindungen der Formel VIII acyliert, 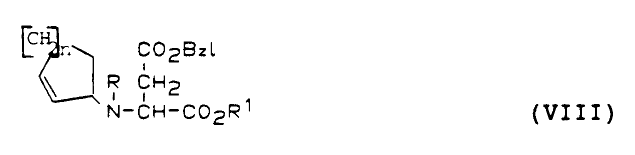
worin n, R und R¹ wie oben definiert sind. Die Acylierung führt man zweckmäßig vorzugsweise in Gegenwart einer Base in einem dipolar aprotischen Lösungsmittel wie Aceton zwischen -20° C und dem Siedepunkt des Reaktionsgemisches, vorzugsweise bei Raumtemperature durch. Als Acylierungsmittel kommen beispielsweise die Chloride der Formel R Cl oder die Anhydride der Formel R₂O in Frage. Geeignete Basen sind tert. Amine wie Triethylamin und anorganische Basen wie K₂CO₃.

Die Verseifung des Diesters VII mit Alkali, vorzugsweise NaOH in DMF bei Raumtemperatur liefert die Verbindung der Formel IX 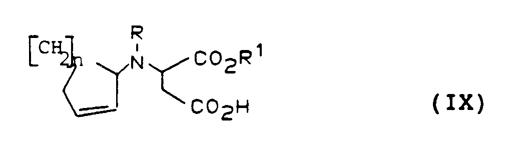
in der n, R und R¹ wie oben definiert sind.

Diese aktiviert man bei -30 bis 0° C in einem dipolar aprotischen Lösungsmittel wie Tetrahydrofuran durch Zugabe eines Chlorameisensäureniederalkylesters, vorzugsweise Chlorameisensäureisobutylesters und einer Base wie N-Methylmorpholin, wobei sich intermediäre Verbindungen der Formel X bilden, 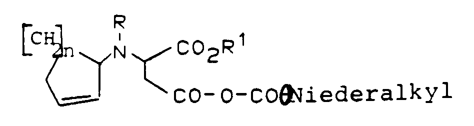
in der n, R und R¹ obige Bedeutung haben.

Unter Beibehaltung der Temperature setzt man nun das Alkalisalz vorzugsweise das Natriumsalz von Thiohydroxamsäuren vorzugsweise von N-Hydroxy-2H-pyridin-2-thion zu, wobei die Verbindung der Formel II bildet.

Neben N-Hydroxy-2H-pyridin-2-thion 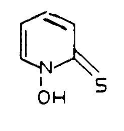
sind beispielsweise auch die folgenden Thiohydroxamsäuren geeignet: 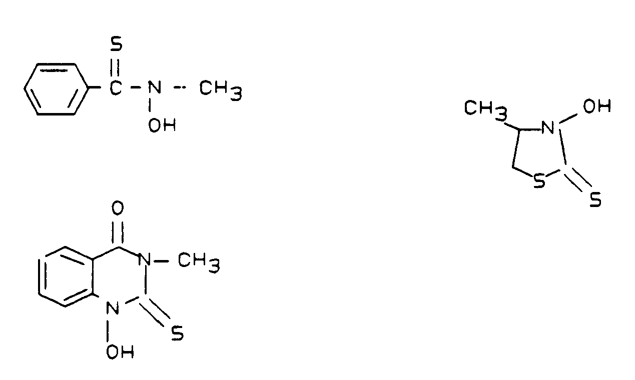
Die Erfindung betrifft auch Verbindungen der Formel III, 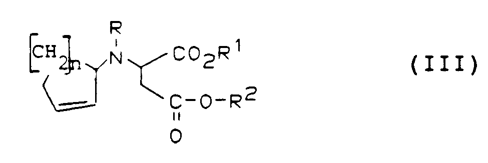
in welcher n, R und R¹ wie oben definiert sind und R² insbesondere 2-Thioxo-2H-[1]pyridyl oder (C₁-C₆)-Alkoxycarbonyl bedeutet, die Zwischenprodukte beim oben genannten Verfahren sind.

Des weiteren betrifft die Erfindung die Verwendung von Verbindungen der Formel I bei enem Verfahren zur Herstellung einer Verbindung der Formel IV, in welcher
R³ Wasserstoff bedeutet oder wie R definiert ist und
R⁴ Wasserstoff bedeutet oder wie R¹ definiert ist,
durch Behandlung einer Verbindung der Formel I mit Raney-Nickel in einem geeigneten Lösungsmittel vorzugsweise einem niederen Alkohol, Wasser oder Dioxan, wobei anschließend gegenbenfalls 1 oder 2 der Reste R³ und/oder R⁴, welche nicht Wasserstoff bedeuten, mit Hilfe von Säuren und/oder Basen und/oder hydrogenolytisch durch Wasserstoff ersetzt werden und wobei gegebenenfalls eine Verbindung der Formel IV durch Verestern oder Umestern in einem Ester der Formel IV überführt wird, worin R⁴ wie R definiert ist. Bromsubstituenten am R=Aroyl werden dabei durch Wasserstoff ersetzt. Unter einem niederen Alkohol werden aliphatische Alkohole mit 1 bis 4 C-Atomen verstanden; bevorzugt ist Ethanol. Die Reaktion wird vorzugsweise zwischen -20° C und dem Siedepunkt der Reaktionsmischung, insbesondere zwischen 10 und 40° C durchgeführt. Neben Raney-Nickel können auch andere Entschwefelungsmittel, wie z.B. Nickelborid Verwendung finden.

Die nachstehenden Beispiele dienen zur Erläuterung der Erfindung, ohne daß diese darauf beschränkt wäre.

### Beispiel 1

### N-(2-Cyclohexen-1-yl)-L-asparaginsäure-(β-benzylester)-α-tert. butylester

In einem 250 ml-Kolben mit 4,324 g (15,5 mMol) L-Asparaginsäure-(β-benzylester)-α-tert.butylester und 6,417 g (46,5 mMol) Kaliumcarbonat in 65 ml wasserfreiem Acetonitril gibt man eine Lösung von 3,22 g (20 mMol) 3-Bromcyclohexen in 20 ml Acetonitril. Man rührt die Mischung heftig bei Zimmertemperatur 48 Std. lang. Das K₂CO₃ wird abfiltriert und mit reichlich Acetonitril gespült. Das Lösungsmittel wird bei verringertem Druck verdampft, und das Amin über Kieselgel gereinigt (Laufmittel: Diethylether/Hexan 1:1).
Ausbeute: 5,02 g (89 % d.Th.) eines leicht gelblichen Öles, 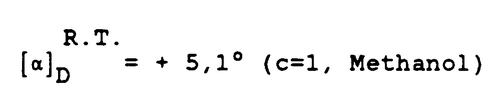
IR:ν = 3320; 1735; 1500; 1455 cm⁻¹
¹H-NMR: δ[ppm]=
1,49 (9H, S, OtBu); 1,24 - 2,12 (7H, m, -(CH₂)₃- + NH) 3,12 (1H, m, -CH-NH-); 2,63 (2H, d, J=6Hz, -CH₂-COOBzl); 3,62 (1H, t, J=6Hz, -CH t); 5,09 (2H, s, -CH₂-Ph); 5,6 (2H, m, -CH=CH-); 7,28 (5H, s, Ph).

| Elementaranalyse (C₂₁H₂₉NO₄ : 359): | | | | |
|---|---|---|---|---|
| berechnet: | % | C 70,17 | H 8,13 | O 17,80 |
| gefunden: | % | C 69,99 | H 7,99 | O 17,86 |

Massenspektrum: m/Z: 360 (M⁺+1) 259 (M⁺-COOtBu). (R.T. beim optischen Drehwert steht für "Raumtemperatur").

### Beispiel 2

### N-Acetyl-N-(2-Cyclohexen-1-yl)-L-asparaginsäure(β-benzylester-α-tert.butylester

Man löst 1,1 g (3,06 mMol) des Amins aus Beispiel 1 in 12 ml Aceton und fügt 1,1 g (3,06 mMol) Kaliumcarbonat zu. Dann gibt man 0,43 ml Acetylchlorid (2 Äquiv.) in 3 ml Äther gelöst zu und rührt die Mischung kräftig 2 Tage bei Zimmertemperatur. Man filtriert das Carbonat ab, spült es mit reichlich Aceton nach und verdampft das Lösungsmittel bei verringertem Druck. Das polare Amid wird über eine Kievelgelsäule gereinigt. (Laufmittel: Diethylether/Hexan 1:1, dann 2:1).
Ausbeute: 1,19 g (97 % d.Th.) eines farblosen Öles; 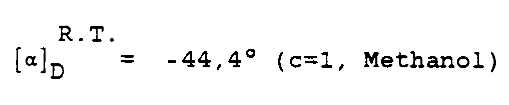
IR: ν= 1740; 1730; 1675; 1650; 1500 cm⁻¹
¹HR-NMR: δ[ppm]=
1,41 (9H, s, OtBu); 1,85 (6H, m) 2,06 (3H, s, COCH₃); 3,62 (2H, m, -CH₂-COOBzl); 4,19 (1H, dd, J₁ = 9Hz, J₂=3Hz, CH₂) 4,35 (1H, m, -CH-N); 5,1 (2H, s, -CH₂-Ph); 5,82 (2H, m, -CH=CH-); 7,3 (5H, s, -Ph).

| Elementaranalyse (C₂₃H₃₁O₅N: 401) | | | | |
|---|---|---|---|---|
| berechnet: | % | C 68,80 | H 7,78 | O 19,93 |
| gefunden: | % | C 68,71 | H 7,85 | O 19,95 |

Massenspektrum
m/z = 401 (M⁺); 358 (M⁺ - COCH₃)

### Beispiel 3

### N-Acetyl-N-(2-cyclohexen-1-yl)-L-asparaginsäure-α-tert.butylester

1,3 g (3,24 mMol) des Amids aus Beispiel 2 werden in 5 ml DMF gelöst und mit 2 ml (1,2 Äquiv.) einer 2N Natronlauge während 2 ½ Tagen bei Raumtemperatur verseift. Das Lösungsmittel verdampft man, und den Rückstand löst man in 2 ml Wasser auf. Man sollte sich vergewissern, daß der pH-Wert alkalisch ist. Diese wäßrige Phase wird mit Äther gewaschen, um den Benzylalkohol zu entfernen und dann mit fester Zitronensäure bis zu einem pH-Wert von 4 sauer gestellt. Dann extrahiert man mit Ethylacetat. Die organische Phase wäscht man mit gesättigter wäßriger NaCl-Lösung, trocknet sie über Na₂SO₄, filtriert und rotiert ein. Die so erhaltene Säure reinigt man über eine Kieselgelsäule (40 g SiO₂ 60-200µm; Laufmittel CH₂Cl₂/Methanol 98:2)
Ausbeute: 910 mg (90 % d.Th.) eines Schaumes, 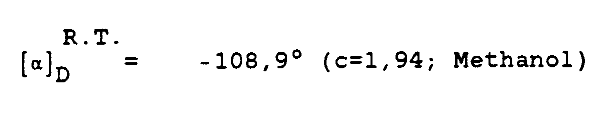
IR: ν= 3350; 1740; 1700 cm⁻¹
¹H-NMR: δ [ppm] =
1,42 (9H, s, OtBu); 1,92 (6H, m); 2,12 (3H, s, -COCH₃); 3,56 (2H, m, -CH₂-); 4,16 (1H, dd, J₁= 9Hz, J₂= 3Hz, -CH₂); 4,35 (1H, m, -CH-N-); 5,51-5,86 (2H, m, -CH=CH-)
Massenspektrum
m/z: 311 (M⁺); 212 (M⁺- COOtBu); 268 (M⁺ - 43)

### Beispiel 4

### N-(p-Brombenzoyl)-N-(2-cyclohexen-1-yl)-L-asparaginsäure(β-benzylester-α-tert.butylester

Man versetzt eine Suspension von 3,86 g (28 mMol) Kaliumcarbonat in 32 ml Aceton mit 2,5 g (6,9 mMol) L-Asparaginsäure(β-benzylester)-α-tert.butylester und fügt eine Lösung von 2,26 g (10,3 mMol) p-Brombenzoesäurechlorid in 10 ml Äther zu. Dann rührt man die Mischung kräftig 2 Tage lang bei Zimmertemperatur, filtriert das Carbonat ab, spült mit reichlich Aceton nach und verdampft das Lösungsmittel bei verringertem Druck. Das zurückbleibende Öl reinigt man über eine Kieselgelsäule (Laufmittel: Diethylether/Hexan 1:3)
Ausbeute: 3,046 g (81 % d.Th.); Fp: 100-101° C (aus Diethylether/Hexan); 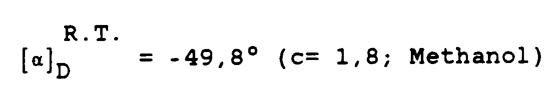
IR (Nujol): ν= 1730; 1635; 1590; 1420 cm⁻¹
¹H-MNR (200 MHz); δ [ppm]=
1,45 (9H, s, OtBu); 1,32-2,15 (6H, m, -CH₂-); 2,47 (1H, dt, J₁=3Hz; J₂=12Hz; -CH₂-); 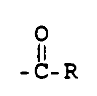
3,73 (1H, m, 1H von, CH₂); 4,23 (2H, m, -CH-N-CH); 5,13 (2H, m, -CH₂-Ph); 5,7 (2H, m, -CH=CH-); 7,28 (9H, m, Ar).

| Elementaranalyse (C₂₈ H₃₂ NO₅ Br : 542) | | | | | |
|---|---|---|---|---|---|
| berechnet: | % | C 61,99 | H 5,94 | N 2,59 | O 14,74 |
| gefunden: | % | C 61,87 | H 5,78 | N 2,46 | O 14,47 |

### Beispiel 5

### N-(p-Brombenzoyl)-N-(2-cyclohexen-1-yl)-L-asparaginsäure-α-tert.butylester

Man verseift 1 g (1,8 mMol) des Amids aus Beispiel 4 durch Zugabe von 2 ml (1,1 Äquiv.) 1 N Natronlauge, rührt die Mischung 1 Tag bei Zimmertemperatur und verdampft dann das Dioxan. Die wäßrige Phase wäscht man mit Äther, stellt ihren pH-Wert mit fester Zitronensäure auf 4 und extrahiert dann mit Ethylacetat.

Die so erhaltene Säure reinigt man über ein Kieselgelsäule (30 g SiO₂ 70-200 µm; Laufmittel (CH₂Cl₂/Methanol 98:2)
Ausbeute: 685 mg (84 % d.Th.) eines Schaums.
IR: ν= 3450; 1730; 1680; 1585 cm⁻¹
¹H-NMR: δ[ppm ] =
1,47 (9H, s, OtBu); 1,3-2,62 (6H, m); 3,65 (2H, m, -CH₂-COOH); 4,22 (2H, m, -CH-N-CH-); 5,84 (2H, m, -CH=CH-); 7,16 (2H, d, J=8Hz); 7,47 (2H, d, J=8Hz, Ar); 8,1 (1H, s, -COOH).

Die Mikroanalyse erfolgte bei dem Dicyclohexylaminsalz, desgleichen wurden der Schmelzpunkt und das Drehvermögen des Dicyclohexylaminsalzes bestimmt.

| Elementaranalyse: (C₃₃H₄₉ N₂O₅ Br : 633) | | | | | |
|---|---|---|---|---|---|
| berechnet: | % | C 62,55 | H 7,79 | N 4,42 | O 12,62 |
| gefunden : | % | C62,32 | H 7,68 | H 4,25 | O 12,47 |

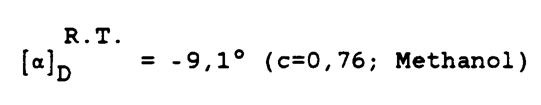

Fp: 156-157° C [aus Ethylacetat/Petrolether].

### Beispiel 6

### 1-Acetyl-4-(2'-pyridyl)-mercapto-perhydro-indol-2-L-carbonsäure-tert.butylester

Man versetzt eine Lösung von 777 mg (2,5 mMol) der Säure aus Beispiel 3 in 13 ml THF unter Rühren und Argon bei -15° C mit 0,28 ml (2,5 mMol) N-Methylmorpholin und 0,36 ml (2,5 mMol) Chlorameisensäureisobutylester. Nach fünfminütiger Aktivierungszeit gibt man 484 mg (1,3 Äquiv.) des Natriumsalzes von N-Hydroxy-2H-pyridin-2-thion zu und rührt die Mischung 1,5 Stunden bei -15° C unter Argon und Lichtausschluß. Dann verdünnt man mit 20 ml THF und läßt sie 2 Stunden bei Zimmertemperatur stehen. Das THF verdampft man bei verringertem Druck, und den Rückstand reinigt man über Kieselgel (Laufmittel: Diethylether/Hexan 2:1, 3:1, dann 4:1 und, sobald das erste Diasterioisomere abgetrennt ist, muß man Diethylether als Laufmittel benutzen).

| | | |
|---|---|---|
| Bilanz: | Diastereomer 1 | 263 mg |
| | Mischung aus Diastereomer1 und 2 | 160 mg |
| | Diastereomer 2 | 151 mg |
| | Gesamtausbeute | 574 mg (56 % d.Th.) |

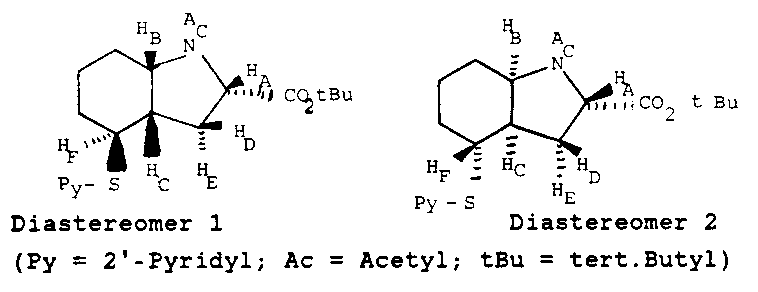

Die 160 mg der obigen Diastereomerenmischung wurden mittels präparativer Dünnschichtchromatographie (SiO₂; Laufmittel: Diethylether/Hexan 1:1) getrennt, wobei weitere 17 mg Diastereomer 1 und 125 mg Diastereomer 2 erhalten wurden.

### Diastereomer 1

Leicht gelbes Öl; 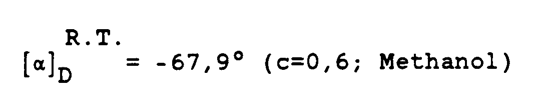
IR: ν= 1740; 1720; 1640; 1585; 1560 cm⁻¹
¹H-NMR (200 MHz): δ[ppm] =
1,53 (9H, s, OtBu); 2,18 (3H, s, -COCH₃); 1,43-2,35 (7H, m, -CH₂-); 2,5 (1H, m, H_{D} oder H_{E}, 2,72 (1H,m, H_{C}); 4,15 (1H, m, H_{B}); 4,45 (1H, t, J=10Hz, H_{F}); 4,58 (1H, m, H_{A}); 7,23-7,93 (3H, m, -S-Py); 8,8 (1H, m, -S-Py)
Massenspektrum
m/z = 376 (M⁺), 320 (M⁺- 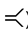 ), 276 (M⁺-COOtBu) 265 (M⁺-S-Py)

### Diastereomer 2:

Fp: 163-165° C [aus Diethglether /Hexan]; 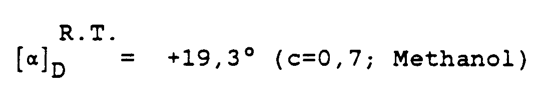
¹H-NMR (400 MHz): δ[ppm] =
1,5 (9H, s, OtBu); 2,12 (3H, s, -COCH₃); 1,16-2,29 (7H, m); 2,39 (1H, m, H_{D} oder H_{E}); 2,63 (1H, m, H_{C}); 3,97 (1H, m, H_{B}); 4,26 (1H, t, J=9Hz, H_{F}); 4,36 (1H, m, H_{A}); 6,88-7,58 (3H, m, S-Py); 8,38 (1H, m).
IR: ν= 1740; 1720; 1640; 1585; 1560 cm⁻¹
Massenspektrum:
m/z= 376 (M⁺), 320 (M⁺- 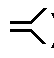 ), 276 (M⁺-COOtBu), 265 (M⁺-S-Py)

### Beispiel 7

### 1-(p-Brombenzoyl)-4-[(2'-pyridyl)-mercapto]-perhydroindol-2-L-carbonsäure-tert.butylester

Ausgehend von 600 mg (1,3 mMol) der Säure aus Beispiel 5 wurde die Titelbindung in analoger Weise zu Beispiel 6 hergestellt.

Die säulenchromatographische Trennung (SiO₂, Laufmittel Diethylether/Hexan 1:2, später 1:1) führte zu einer Auftrennung der Diastereomeren.

| | | |
|---|---|---|
| Bilanz: | Diastereomer 1' | 205 mg |
| | Mischung aus Diastereomer 1' und 2' | 135 mg |
| | Diastereomer 2' | 125 mg |
| | Gesamtausbeute | 465 mg (69 % d.Th.) |

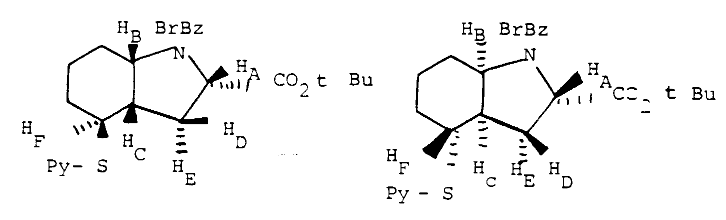

### Diastereomer 1':

Fp: 164-165° C [aus Ethanol];
[α]_{D} = -170,0° (c=1,09; Methanol)
IR: ν= 1740; 1640; 1600; 1580; 1560; 1450 cm⁻¹
¹H-NMR (200 MHz): δ [ppm] =
1,48 (9H, m); 1,1-2,33 (7H, m, -CH₂); 2,48 (2H, m, H_{C} + H_{D} oder( H_{E})); 3,76 (1H, m, H_{B}); 4,31 (1H, m, H_{F})); 4,45 (1H, m, H_{A})); 6,86-7,68 (8H, Ar); 8,36 (1H, m).

| Elementaranalyse (C₂₅H₂₉ N₂O₃ Br :517) | | | | | |
|---|---|---|---|---|---|
| berechnet: | % | C 58,01 | H 5,64 | N 5,44 | O 9,27 |
| gefunden: | % | C 57,88 | H 5,48 | N 5,49 | O 9,08 |

### Diastereomer 2':

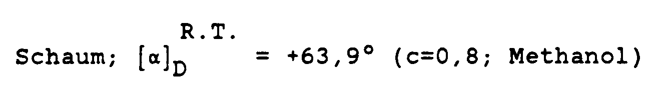

¹H-NMR (400 MHz): δ [ppm] =
1,44 (9H, s, -OtBu); 0,8-1,91 (6H, m, -(CH₂)₃-); 2,09 (1H, dd, J_{EA}=0, J_{ED}=13Hz, J_{EC}=7Hz, H_{E}); 2,36 (1H, dt, J_{DE}=J_{DC}=13Hz, J_{DA}=10Hz, H_{D}); 2,83 (1H, m, H_{C}); 4,03 (1H, m, H_{B}); 4,27 (1H, m, H_{F}); 4,6 (1H, d, J_{DA}=10Hz, H_{A}); 6,90-7,68 (7H, m); 8,32 (1H, m).
Massenspektrum:
m/z: 515, 517, 518 (M⁺), 461 (M⁺-=〈 ) 317 (M⁺-COOtBu), 406 (M⁺-S-Py).

### Beispiel 8:

### cis, exo-N-Benzoyl-perhydroindol-2-L-carbonsäure-tert. butylester

150 mg (0,29 mMol) Diastereomer 1' aus Beispiel 7 werden in 2 ml absolutem Ethanol gelöst und bei Raumtemperatur über Nacht mit Raney-Nickel (®Prolabo, 50 %ig in Wasser) reduziert. Anschließend filtriert man ab, spült den Katalysator mit reichlich Ethanol-Wasser-Gemisch nach und rotiert das Lösungsmittel ab.
Ausbeute: 100 mg Rohprodukt.

### Beispiel 9:

### cis, exo-N-Benzoyl-perhydroindol-2-L-carbonsäure

Das Rohprodukt aus Beispiel 8 wird in einer Mischung aus 0,2 ml Trifluoressigsäure und 0,2 ml CH₂Cl₂ eine Stunde bei Raumtemperatur hydrolysiert. Anschließend wird eingeengt und der ölige Rückstand (87 mg) mit Pentan gewaschen.

### Beispiel 10:

### cis, exo-N-Benzoyl-perhydroindol-2-L-carbonsäure-ethylester

Man löst die Säure aus Beispiel 9 in 1,2 ml DMF und neutralisiert sie mit 52 mg (0,6 mMol) NaHCO₃. Nun setzt man eine Lösung von 0,1 ml (1,25 mMol) Ethylbromid in 1,2 ml DMF zu und rührt 24 Stunden bei Raumtemperatur. Das DMF wird bei verringertem Druck abgedampft und der Rückstand in 1,5 ml einer 10 %igen wäßrigen Zitronensäurelösung aufgenommen. Die Titelverbindung wird mit Ethylacetat extrahiert und dann chromatographisch gereinigt (präparative DC, SiO₂; Laufmittel: Diethylether/Hexan 1:1).
Ausbeute 57 mg (65 % d.Th.) eines farblosen Öles;
[α]_{D} = -41,1° (c=0,48, Methanol).
IR: ν= 1750; 1640; 1600; 1570 cm⁻¹.
¹H-NMR (400 MHz); δ[ppm] =
1,30 (3H, m, -CO-CH₂-CH₃); 0,85-1,94 (8H, m, -(CH₂)₄); 2,07 (1H, dt, J_{ED}=J_{EC}=13Hz, J_{EA}=8Hz, H_{E}); 2,23 (1H, m, H_{D}); 2,38 (1H, m, H_{C}); 3,57 (1H, m, H_{B}); 4,24 (2H, m, -CO-CH₂-CH₃); 4,62 (1H, dd, J_{AE}=8Hz, J_{AD}=6Hz, H_{A}); 7,31 (2,5H, Ar); 5,87 (2,5 H, Ar).
[Numerierung der H-Atome wie bei Diastereomeren 1ʹ und 2ʹ]
Massenspektrum:
m/z = 301 (M⁺), 237 (M⁺-COOEt), 196 (M⁺-CO-Ph).

### Beispiel 11:

### cis, endo-N-Benzoyl-perhydroindol-2-L-carbonsäureethylester

Man verfährt wie oben unter Beispiel 8-10 unter Verwendung von 120 mg (0,23 mMol) von Diastereomer 2' aus Beispiel 7.
Ausbeute: 46 mg (66 % d.Th.) weiße Kristalle;
Fp: 111-112° C [aus Diethylether/Pentan]; 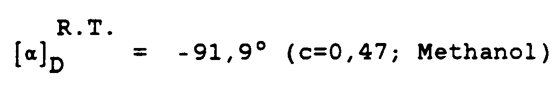
IR: ν= 1750; 1640; 1600; 1570 cm⁻¹.
¹H-NMR (400 MHz): δ[ppm] =
1,3 (3H, t, J=7Hz, CH₃-); 0,83-1,78 (8H, m, -(CH₂-)₄); 1,87 (1H, dd, J_{EA}=0, J_{EB}=13Hz, J_{EC}=6,5Hz, H_{E}); 2,23 (1H, dt, J_{DA}=10Hz, J_{DE}=J_{DC}=13Hz, H_{D}); 2,67 (1H, m, H_{C}); 3,26 (1H, m, H_{B}); 4,22 (2H, q, J=7Hz, -CH₂-CH₃); 4,69 (1H, d, J_{AD}=10Hz, H_{A}); 7,37 (2,5H, m, Ar); 7,48 (2,5H, m, Ar).
[Numerierung der H-Atome wie bei Diasteromeren 1' und 2']
Massenspektrum:
m/z = 301 (M⁺), 237 (M⁺-COOEt), 196 (M⁺-COPh).

### Beispiel 12:

### cis,exo-N-Acetyl-perhydroindol-2-L-carbonsäure-tert. butylester

150 mg (0,4 mMol) Diastereomer 1 aus Beispiel 6 werden in 3 ml Ethanol gelöst und über Nacht unter Rühren bei Raumtemperatur mit Raney-Nickel reduziert. Man filtriert vom Nickel ab, spült mit einer Wasser-Ethanol-Mischung nach und engt im Vakuum ein.
Rohausbeute: 130 mg.

### Beispiel 13:

### cis, exo-Perhydroindol-2-L-carbonsäure-Hydrochlorid

Das Rohprodukt aus Beispiel 12 wird mit 4 ml 6 N Salzsäure eine Stunde am Rückfluß gekocht. Nach Einengen im Vakuum bleibt das Hydrochlorid in Form eines Öles zurück.

### Beispiel 14:

### cis, exo-Perhydroindol-2-L-carbonsäureethylester-Hydrochlorid

In eine Lösung des Hydrochlorids aus Beispiel 13 in 3 ml absolutem Ethanol wird bei Raumtemperatur 5 Minuten lang und bei 0° C 10 Minuten lang Chlorwasserstoff eingeleitet. Nach Abdampfen des Ethanols bleibt als Rückstand das Hydrochlorid des Ethylesters (100 mg).

### Beispiel 15:

### cis, exo-N-(p-Brombenzoyl)-perhydroindol-2-L-carbonsäureethylester

Man löst das Ether-Hydrochlorid aus Beispiel 14 in einer Mischung aus 3 ml THF und 1 ml CH₂Cl₂ und gibt bei 0° C 0,07 ml (0,5 mMol) Triethylamin zu. Dann gibt man nacheinander bei 0° C 151 mg (0,75 mMol) p-Brombenzoesäure, 115 mg (0,75 mMol) Hydroxybenzotriazol und 155 mg (0,75 mMol) Dicyclohexylcarbodiimid zu und rührt die Mischung kräftig 24 Stunden bei Raumtemperatur. Man filtriert vom Dicyclohexyl Harnstoff ab, spült mit THF und verdampft das Lösungsmittel.

Man löst den Rückstand in 5 ml Ethylacetat, wäscht die organische Phase nacheinader mit 0,1 N wäßriger NaHCO₃-Lösung, Wasser, 0,5 N Salzsäure, nochmals Wasser und einer gesättigten wäßrigen NaCl-Lösung. Dann trocknet man über Na₂SO₄, filtriert und engt im Vakuum ein. Das so erhaltene Derivat wird nun mittels präparativer Dünnschichtchromatographie gereinigt (SiO₂; Laufmittel Diethylether/Hexan 1:1).
Ausbeute: 62 mg (41 % d.Th.) eines farblosen Öles; 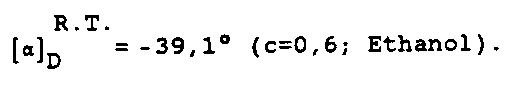
IR: ν= 1745; 1635; 1595; 1490 cm⁻¹.
¹H-NMR (200 MHz): δ [ppm] =
1,3 (3H, m, CH₃); 0,83-1,95 (8H, m, -(CH₂)₄-); 2,17 (1H, m, H_{E}); 2,37 (1H, dt, J_{DC}=J_{DA}=8Hz, J_{DE}=16Hz, H_{D}) 2,71 (1H, m, H_{C}); 3,6 (1H, m, H_{B}); 4,27 (2H, m, -COOCH₂-CH₃); 4,67 (1H, m, H_{A}); 7,37 (2H, d, J=9Hz); 7,6 (2H, d, J=9Hz).
[Numerierung der H-Atome wie bei Diastereomeren 1 und 2]
Massenspektrum:
m/z= 320 (M⁺), 307 (M⁺-COOEt), 196 (M⁺-COAr).

### Beispiel 16:

### cis, endo-N-(p-Brombenzoyl)-perhydroindol-2-L-carbonsäureethylester

Ausgehend von 120 mg (0,32 mMol) Diastereomer 2 aus Beispiel 6 analog den Beispielen 12-15 erhält man 55 mg (45% d.Th.) der Titelverbindung in Form eines Öles. 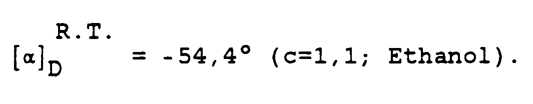
IR: ν= 1745; 1635; 1595; 1495 cm⁻¹.
¹H-NMR (200 MHz): δ [ppm]
1,32 (3H, t, J=7Hz, -Ch₃); 0,83-1,8 (8H, m, -(CH₂)₄-); 1,88 (1H, dd, J_{ED}=13Hz, J_{EC}=6,5Hz, H_{E}); 2,37 (1H, dt, J_{DE}=J_{DC}=13Hz; J_{DA}=9Hz, H_{D}); 2,6 (1H, m, H_{C}); 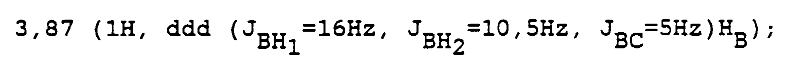
4,2 (2H, q, J=7Hz, -O-CH₂-CH₃); 4,68 (1H, d, J=9Hz, H_{A}); 7,38 (2H, d, J=9Hz); 7,55 (2H, d, J=9Hz).
[Numerierung der H-Atome wie bei Diastereomeren 1 und 2]
Massenspektrum:
m/z= 380 (M⁺), 307 (M⁺-COOEt), 196 (M⁺-COAr).

### Beispiel 17:

### cis, exo-N-Benzoyl-perhydroindol-2-L-carbonsäure-tert. butylester

Man erhält die Titelverbindung ausgehend von Diastereomer 1' von Beispiel 7 durch Behandeln mit Raney-Nickel in Ethanol. Die Reinigung erfolgt mittels präparativer Dünnschichtchromatographie (SiO₂ ; Laufmittel Diethylether/Hexan 1:1).
Fp.: 114-115° C [aus Ethanol]; 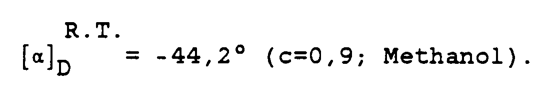
IR: ν = 1740; 1640; 1600; 1420 cm⁻¹
¹H-NMR (200MHz): δ [ppm] =
1,58 (9H, m, OtBu); 0,96-1,96 (8H, m,-(CH₂-)₄); 2,18 (2H, m, H_{D}, H_{E}); 2,43 (1H, m, H_{C}); 3,73 (1H, m, H₈); 4,7 (1H, m, H_{A}); 7,7 (5H, m, Ar).
[Numerierung der H-Atome wie bei Diastereomeren 1' und 2']
Massenspektrum:
m/z = 329 (M⁺), 229 (M⁺-COOtBu), 224 (M⁺-CO-Ph).

### Beispiel 18:

### cis, endo-N-Benzoyl-perhydroindol-2-L-carbonsäure-tert. butylester

Man erhält die Titelverbindung ausgehend von Diastereomer 2' von Beispiel 7 durch Behandeln mit Raney-Nickel in Ethanol. Die Reinigung erfolgt mittels präparativer Dünnschichtchromatographie (SiO₂; Laufmittel Diethylether/Hexan 1:1).
Fp.: 111-112° C [aus Ethanol]; 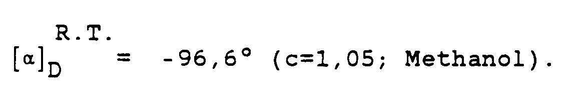
IR: ν= 1740; 1640; 1600; 1420 cm⁻¹.
¹H-NMR (400 MHz): δ [ppm] =
1,5 (9H, s, OtBu); 0,82-1,7 (8H, m, -(CH₂)₄); 1,84 (1H, dd, J_{EA}=0, J_{ED}=13Hz, J_{EC}=6,5Hz, H_{E}); 2,31 (1H, dt, J_{DA}=10Hz, J_{DE}=J_{DC}=13Hz, H_{D}); 2,67 (1H, m, H_{C}); 3,86 (1H, m, H_{B}); 4,56 (1H, d, J=10Hz, H_{A}); 7,42 (5H, m, Ar).
[Numerierung der H-Atome wie bei Diastereomeren 1ʹ und 2ʹ]

| Elementaranalyse (C₂₀ H₂₇ NO₃): | | | | |
|---|---|---|---|---|
| berechnet: | % | C 72,91 | H 8,26 | O 14,57 |
| gefunden: | % | C 72,83 | H 8,38 | O,14,81 |

## Patentansprüche (Patentansprüche für folgende(n) Vertragsstaat(en): AT, BE, CH, DE, FR, GB, IT, LI, LU, NL, SE)

1. Verbindung der Formel (I) 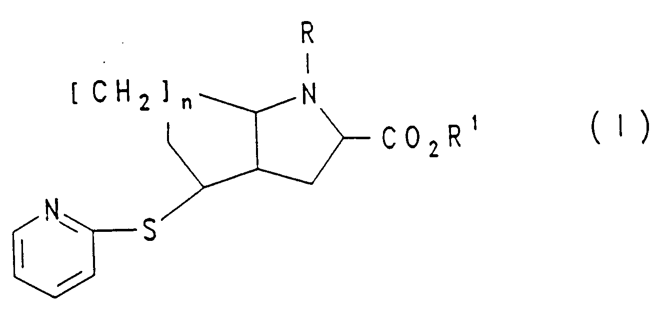 in welcher
n = 1, 2 oder 3
R (C₁-C₆)-Alkanoyl, (C₆-C₁₀)-Aryl-(C₁-C₄)-alkanoyl, (C₆-C₁₀)-Aroyl, (C₁-C₆)-Alkoxycarbonyl oder (C₇-C₁₁)-Aralkyloxycarbonyl, oder eine Schutzgruppe vom Urethantyp aus der Gruppe 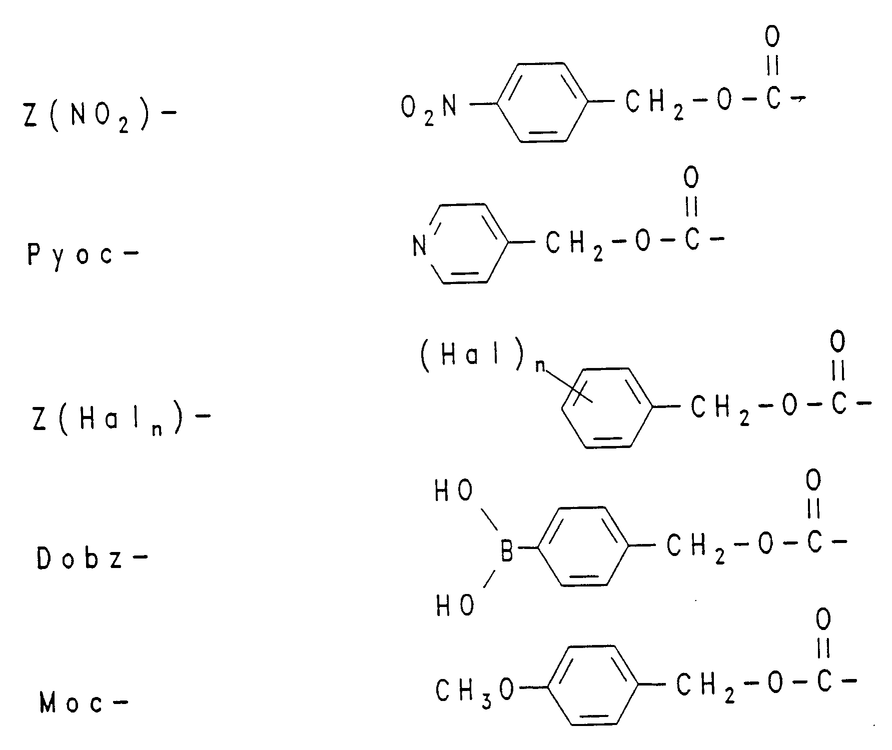 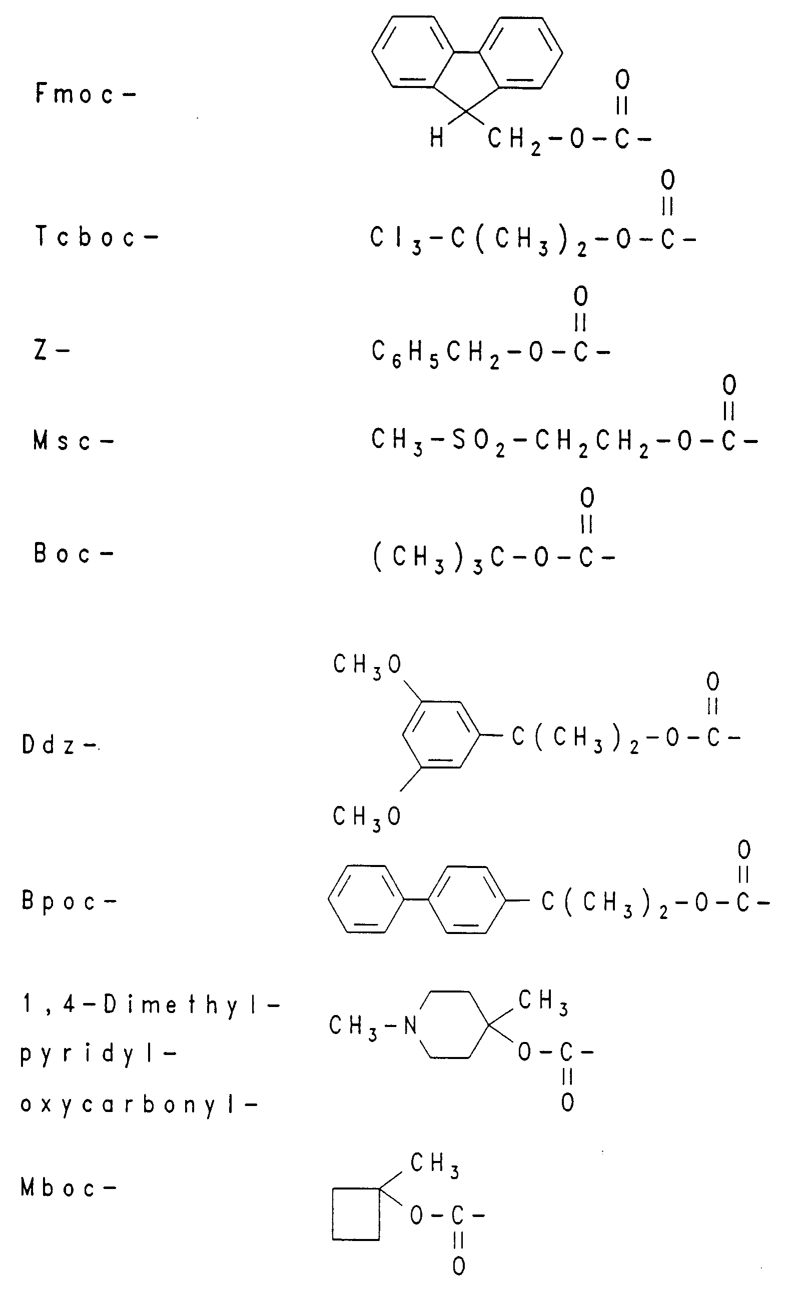 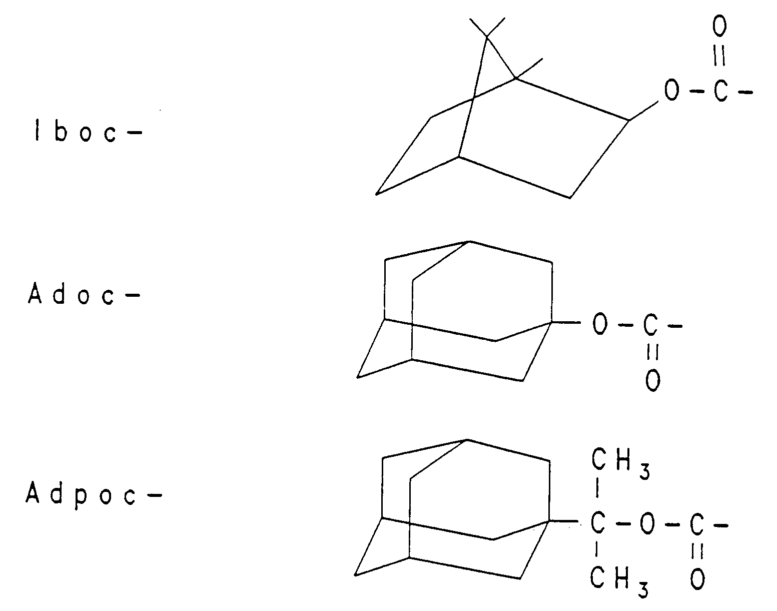
R¹ (C₁-C₆)-Alkyl, (C₃-C₇)-Cycloalkyl, (C₇-C₁₁)-Aralkyl oder eine andere Carboxylschutzgruppe bedeuten.

2. Verbindung der Formel I gemäß Anspruch 1, in welcher die Wasserstoffatome an den Brückenkopf-Kohlenstoffatomen 3a und (5+n)a cis-konfiguriert sind.

3. Verbindung der Formel I gemäß Anspruch 1 oder 2 in welcher das Kohlenstoffatom in Position 2 die S-Konfiguration aufweist.

4. Verfahren zur Herstellung einer Verbindung der Formel I gemäß Anspruch 1, dadurch gekennzeichnet, daß man eine Verbindung der Formel II 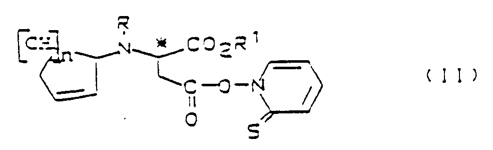 in welcher n, R und R¹ wie im Anspruch 1 definiert sind, durch Erwärmen auf 40-190°C, in einem geeigneten Lösungsmittel oder auch ohne Lösungsmittel gegebenenfalls in Gegenwart eines Radikalinitiators radikalisch decarboxyliert.

5. Verbindung der Formel III 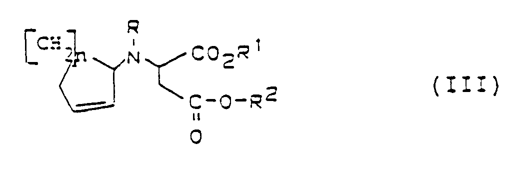 in welcher n, R und R¹ wie im Anspruch 1 definiert sind und
R² 2-Thioxo-2H-[1]pyridyl oder
(C₁-C₆)-Alkoxycarbonyl bedeutet.

6. Verwendung einer Verbindung der Formel I gemäß einem oder mehrere der Ansprüche 1-3 bei einem Verfahren zur Herstellung einer Verbindung der Formel IV 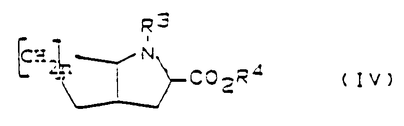 in welcher
R³ Wasserstoff bedeutet oder wie R im Anspruch 1 definiert ist und
R⁴ Wasserstoff bedeutet oder wie R¹ im Anspruch 1 definiert ist,
durch Behandlung einer Verbindung der Formel I mit Raney-Nickel in einem geeigneten Lösungsmittel, wobei anschließend gegebenenfalls 1 oder 2 der Reste R³ und/oder R⁴, welche nicht Wasserstoff bedeuten, mit Hilfe von Säuren und/oder Basen und/oder hydrogenolytisch durch Wasserstoff ersetzt werden und wobei gegebenenfalls eine Verbindung der Formel IV durch Verestern oder Umestern in einen Ester der Formel IV überführt wird, worin R⁴ wie R im Anspruch 1 definiert ist.

## Patentansprüche (Patentansprüche für folgende(n) Vertragsstaat(en): ES, GR)

1. Verfahren zur Herstellung einer Verbindung der Formel (I) 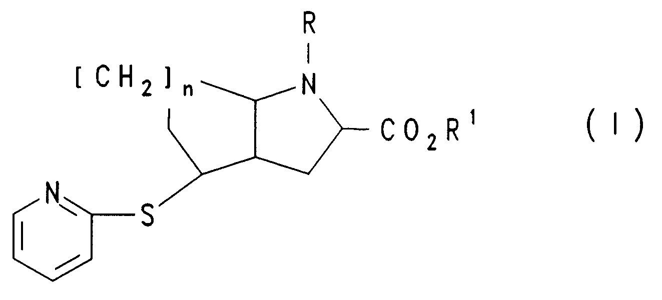 in welcher
n = 1, 2 oder 3
R (C₁-C₆)-Alkanoyl, (C₆-C₁₀)-Aryl-(C₁-C₄)-alkanoyl, (C₆-C₁₀)-Aroyl, (C₁-C₆)-Alkoxycarbonyl oder (C₇-C₁₁)-Aralkyloxycarbonyl, oder eine Schutzgruppe vom Urethantyp aus der Gruppe 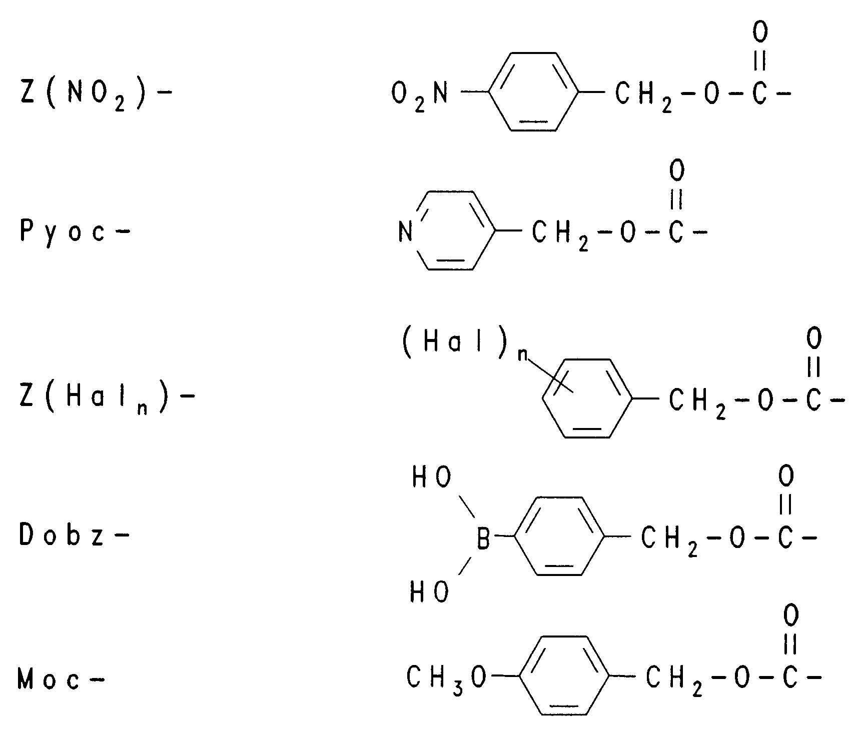 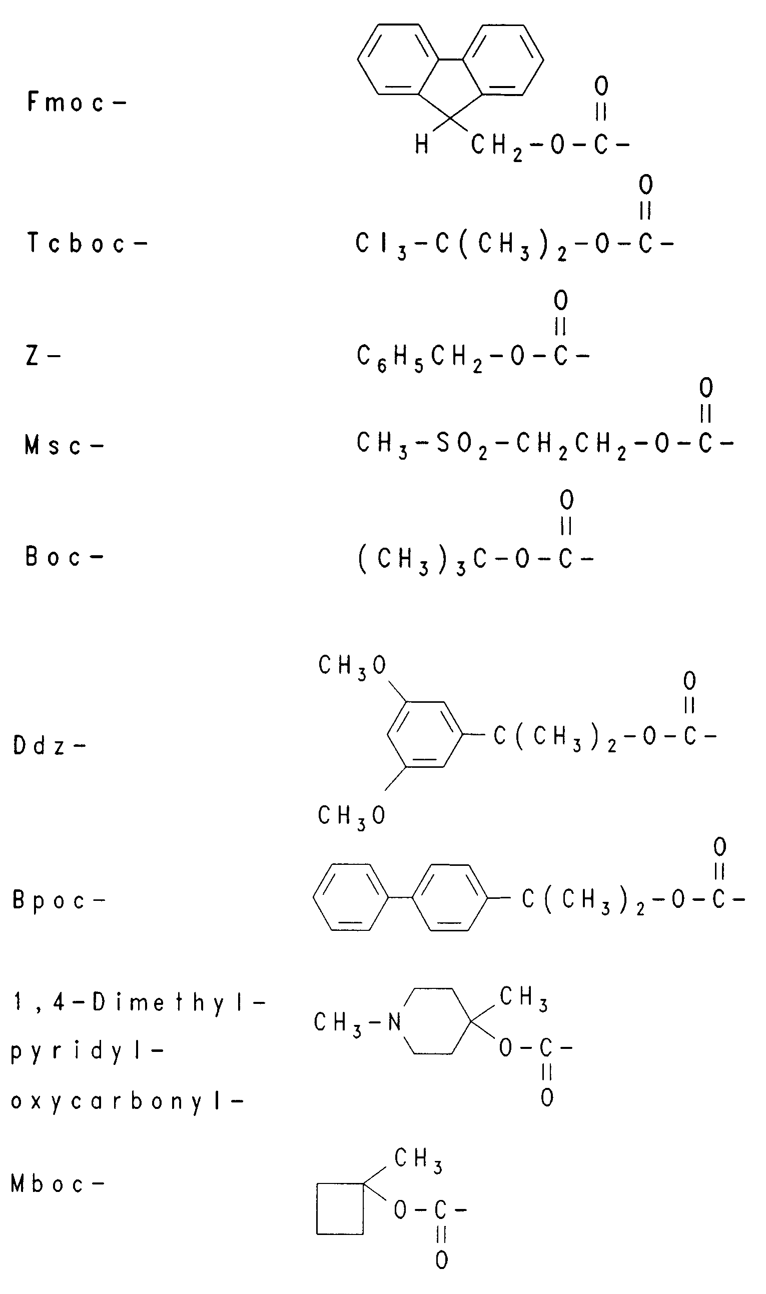 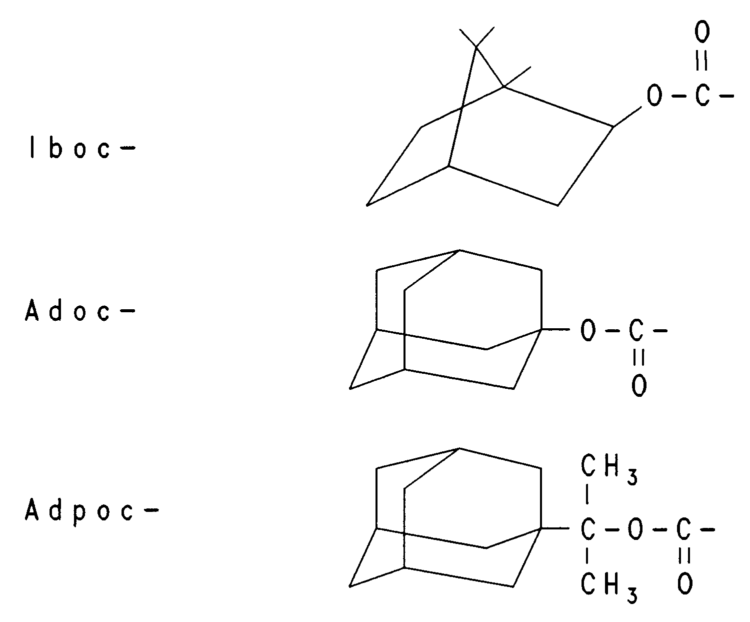
R¹ (C₁-C₆)-Alkyl, (C₃-C₇)-Cycloalkyl, (C₇-C₁₁)-Aralkyl oder eine andere Carboxylschutzgruppe bedeuten,
dadurch gekennzeichnet, daß man eine Verbindung der Formel II 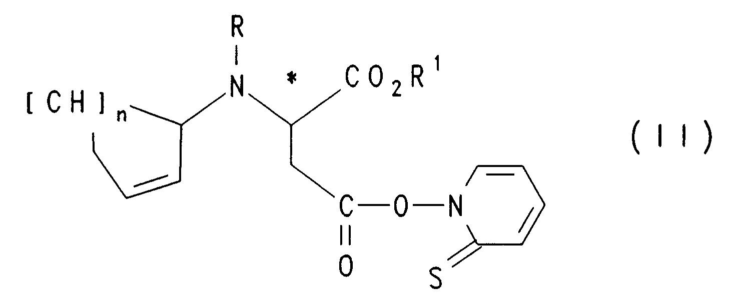 in welcher n, R und R¹ wie oben definiert sind, durch Erwärmen auf 40 bis 190°C in einem geeigneten Lösungsmittel oder auch ohne Lösungsmittel gegebenenfalls in Gegenwart eines Radikalinitiators radikalisch decarboxyliert.

2. Verfahren gemäß Anspruch 1, wobei Verbindungen der Formel (I) hergestellt werden, in welchen die Wasserstoffatome an den Brückkopf-Kohlenstoffatomen 3a und (5+n)a cis-konfiguriert sind.

3. Verfahren gemäß Anspruch 1 oder 2 wobei Verbindugnen der Formel (III) hergestellt werden, in welchen das Kohlenstoffatom in Position 2 die S-Konfiguration aufweist.

4. Verwendung einer Verbindung der Formel I gemäß einem der Ansprüche 1 bis 3 bei einem Verfahren zur Herstellung einer Verbindung der Formel IV 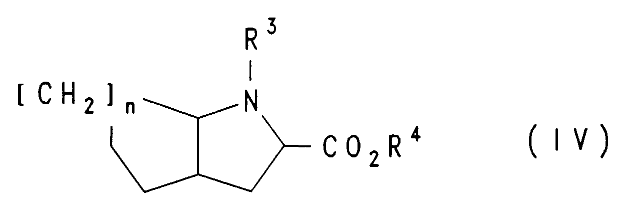 in welcher
R³ Wasserstoff bedeutet oder wie R im Anspruch 1 definiert ist und
R⁴ Wasserstoff bedeutet oder wie R¹ im Anspruch 1 definiert ist,
durch Behandlung einer Verbindung der Formel I mit Raney-Nickel in einem geeigneten Lösungsmittel, wobei anschließend gegebenenfalls 1 oder 2 der Reste R³ und/oder R⁴, welche nicht Wasserstoff bedeuten, mit Hilfe von Säuren und/oder Basen und/oder hydrogenolytisch durch Wasserstoff ersetzt werden und wobei gegebenenfalls eine Verbindung der Formel IV durch Verestern oder Umestern in einen Ester der Formel IV überführt wird, worin R⁴ wie R im Anspruch 1 definiert ist.

## Claims (Claims for the following Contracting State(s): AT, BE, CH, DE, FR, GB, IT, LI, LU, NL, SE)

1. A compound of the formula (I) 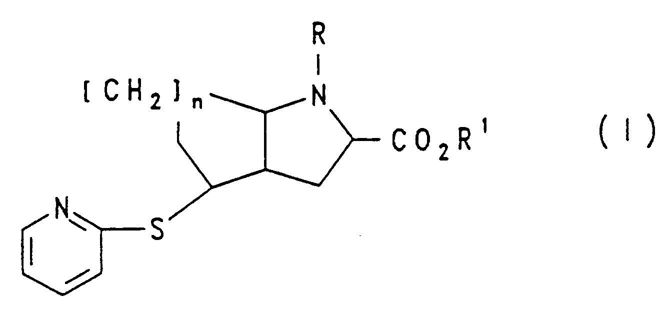 in which
n = 1, 2 or 3,
R denotes (C₁-C₆)-alkanoyl, (C₆-C₁₀)-aryl-(C₁-C₄)-alkanoyl, (C₆-C₁₀)-aroyl, (C₁-C₆)-alkoxycarbonyl or (C₇-C₁₁)-aralkyloxycarbonyl, or denotes a protective group of the urethane type from the group comprising 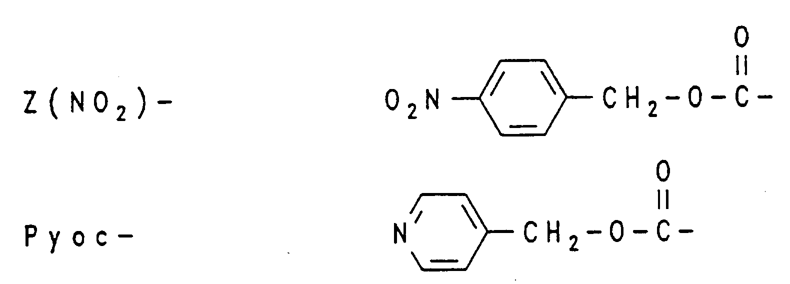 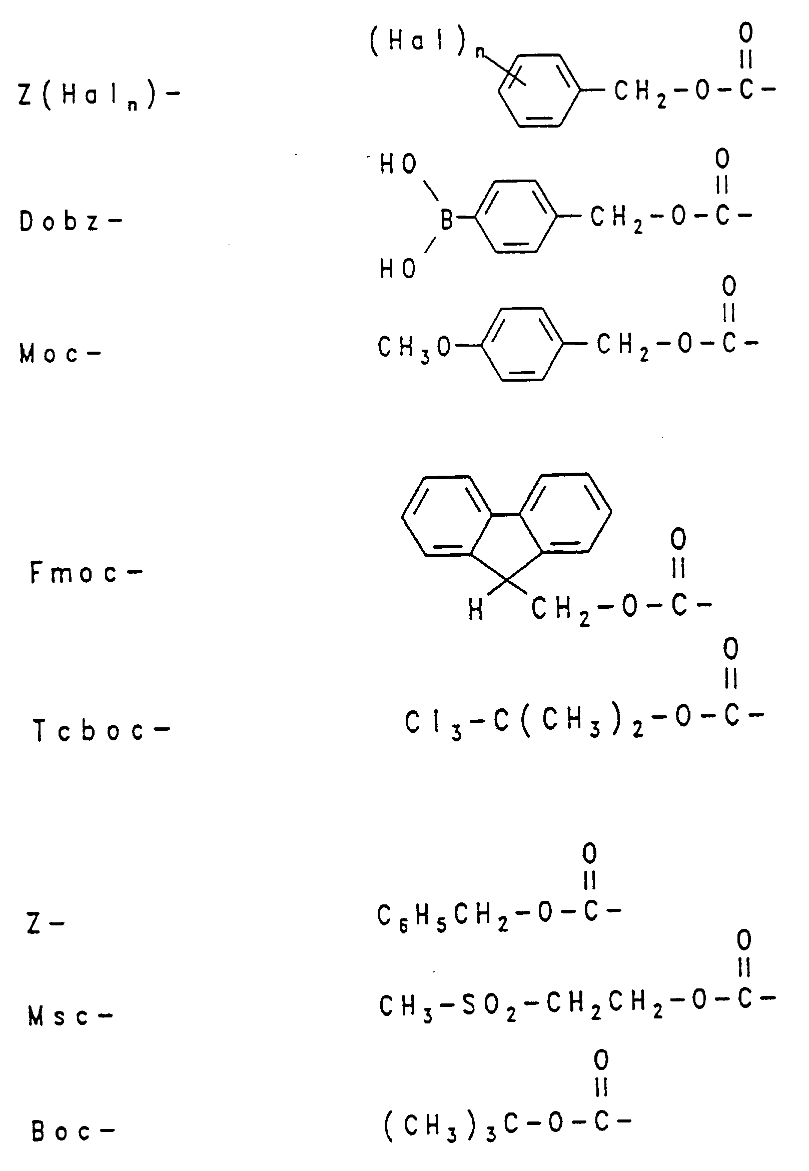 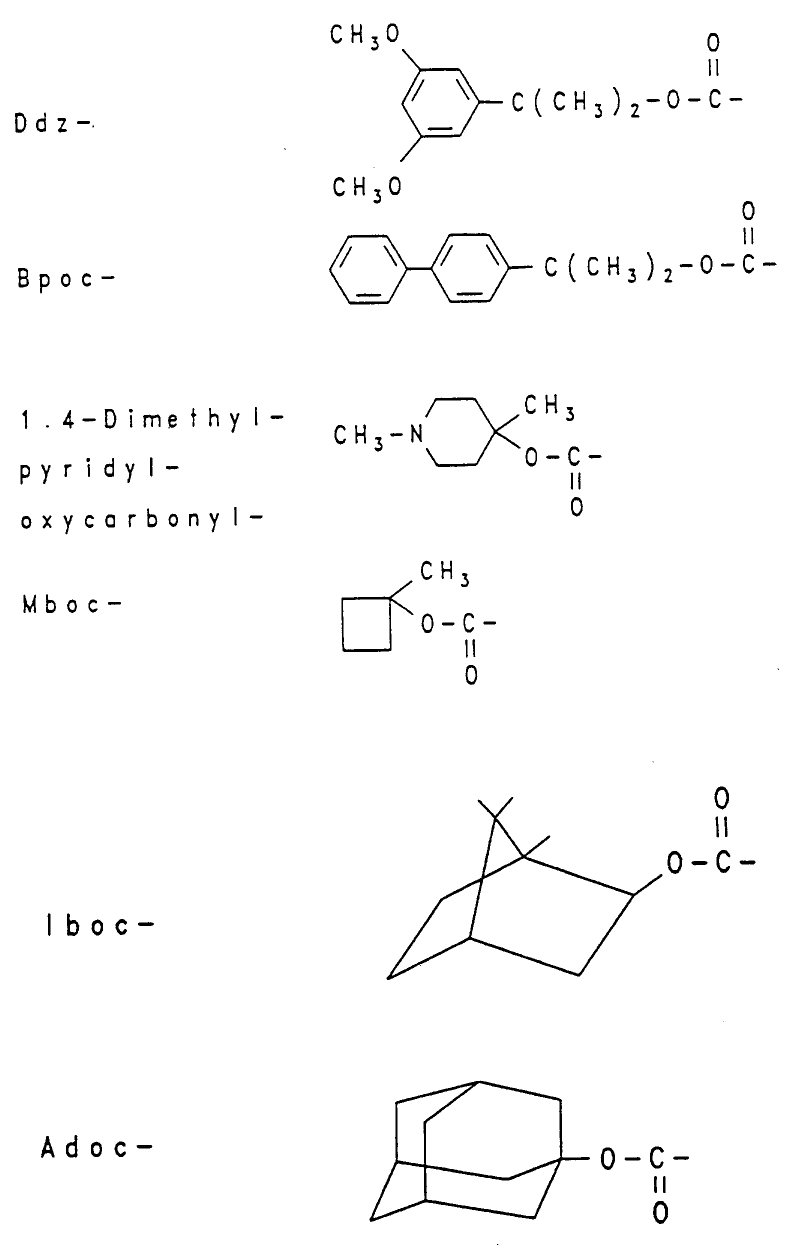 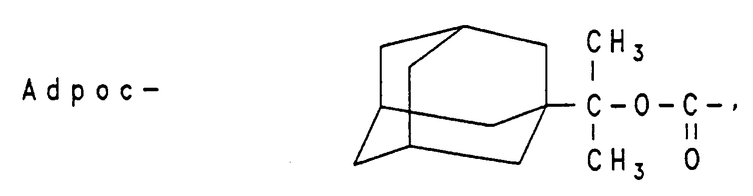 and
R¹ denotes (C₁-C₆)-alkyl, (C₃-C₇)-cycloalkyl, (C₇-C₁₁)-aralkyl or another carboxyl-protecting group.

2. A compound of the formula I as claimed in claim 1, in which the hydrogen atoms on the bridgehead carbon atoms 3a and (5+n)a have a cis configuration.

3. A compound of the formula I as claimed in claim 1 or 2, in which the carbon atom in position 2 has the S configuration.

4. A process for the preparation of a compound of the formula I as claimed in claim 1, which comprises subjecting a compound of the formula II 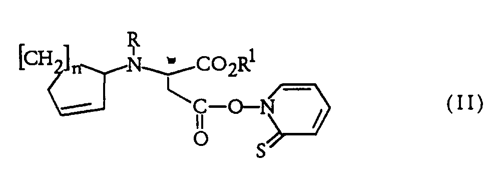 in which n, R and R¹ are as defined in claim 1 to free-radical decarboxylation by heating to 40-190°C in a suitable solvent or else without solvent, in the presence or absence of a free-radical initiator.

5. A compound of the formula III 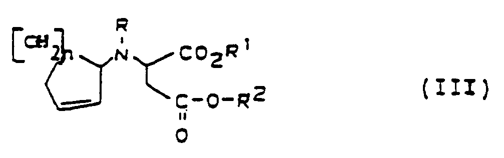 in which n, R and R¹ are as defined in claim 1, and
R² denotes 2-thioxo-2H-[1]pyridyl or (C₁-C₆)-alkoxycarbonyl.

6. The use of a compound of the formula I as claimed in one or more of claims 1-3 in a process for the preparation of a compound of the formula IV 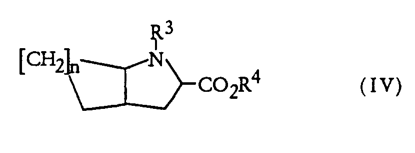 in which
R³ denotes hydrogen or is as defined for R in claim 1, and
R⁴ denotes hydrogen or is as defined for R¹ in claim 1, through treatment of a compound of the formula I with Raney nickel in a suitable solvent, 1 or 2 of the radicals R³ and/or R⁴ which do not denote hydrogen subsequently being replaced, if appropriate, by hydrogen with the aid of acids and/or bases and/or hydrogenolytically, and a compound of the formula IV being converted, if desired, into an ester of the formula IV in which R⁴ is as defined for R in claim 1 by esterification or trans-esterification.

## Claims (Claims for the following Contracting State(s): ES, GR)

1. A process for the preparation of a compound of the formula (I) 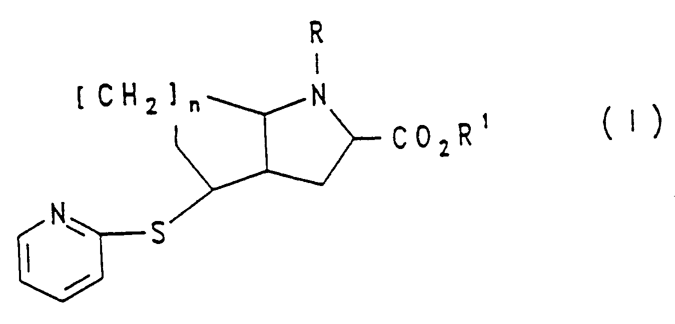 in which
n = 1, 2 or 3,
R denotes (C₁-C₆)-alkanoyl, (C₆-C₁₀)-aryl-(C₁-C₄)-alkanoyl, (C₆-C₁₀)-aroyl, (C₁-C₆)-alkoxycarbonyl or (C₇-C₁₁)-aralkyloxycarbonyl, or denotes a protective group of the urethane type from the group comprising 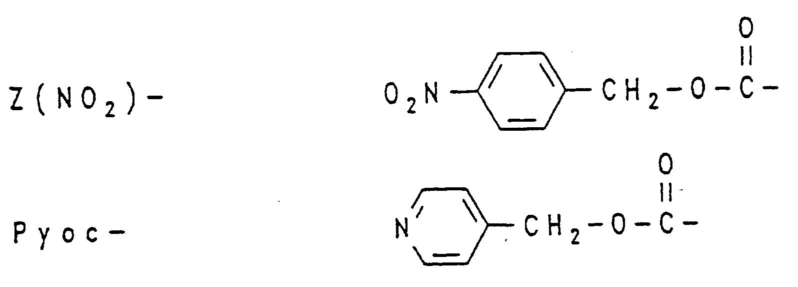 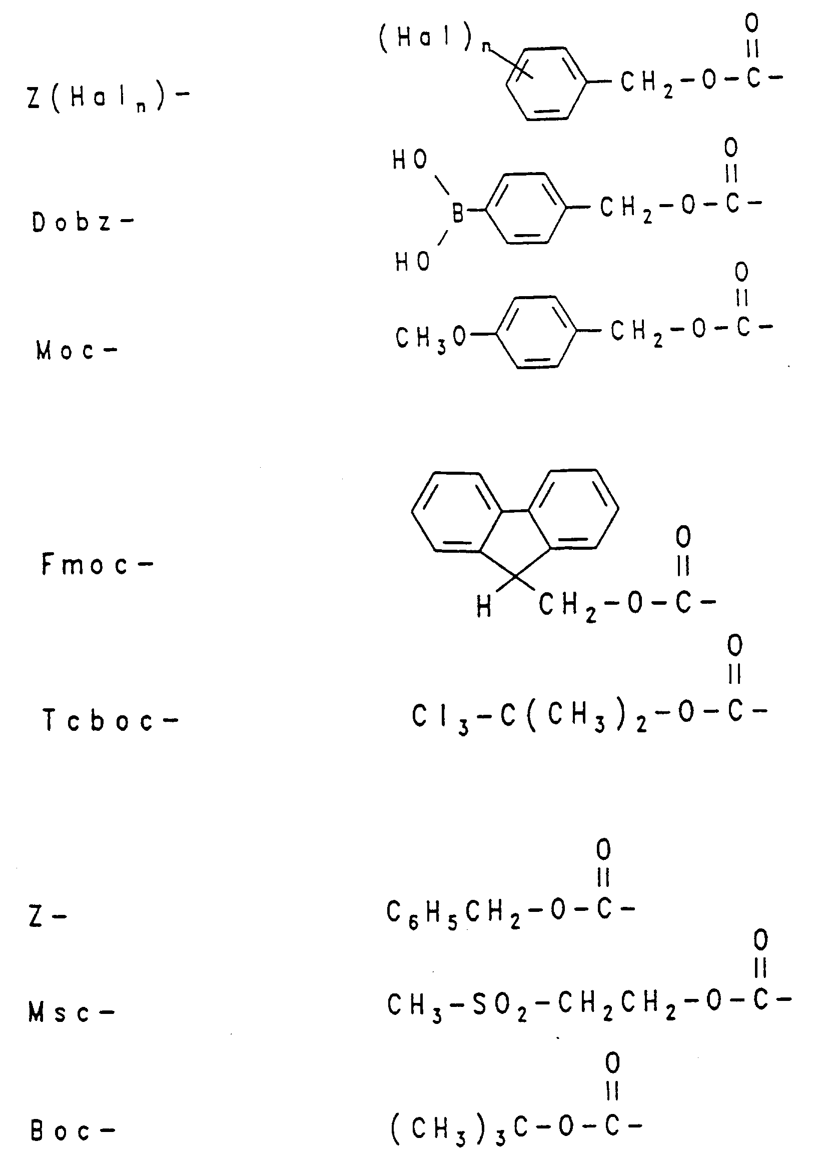 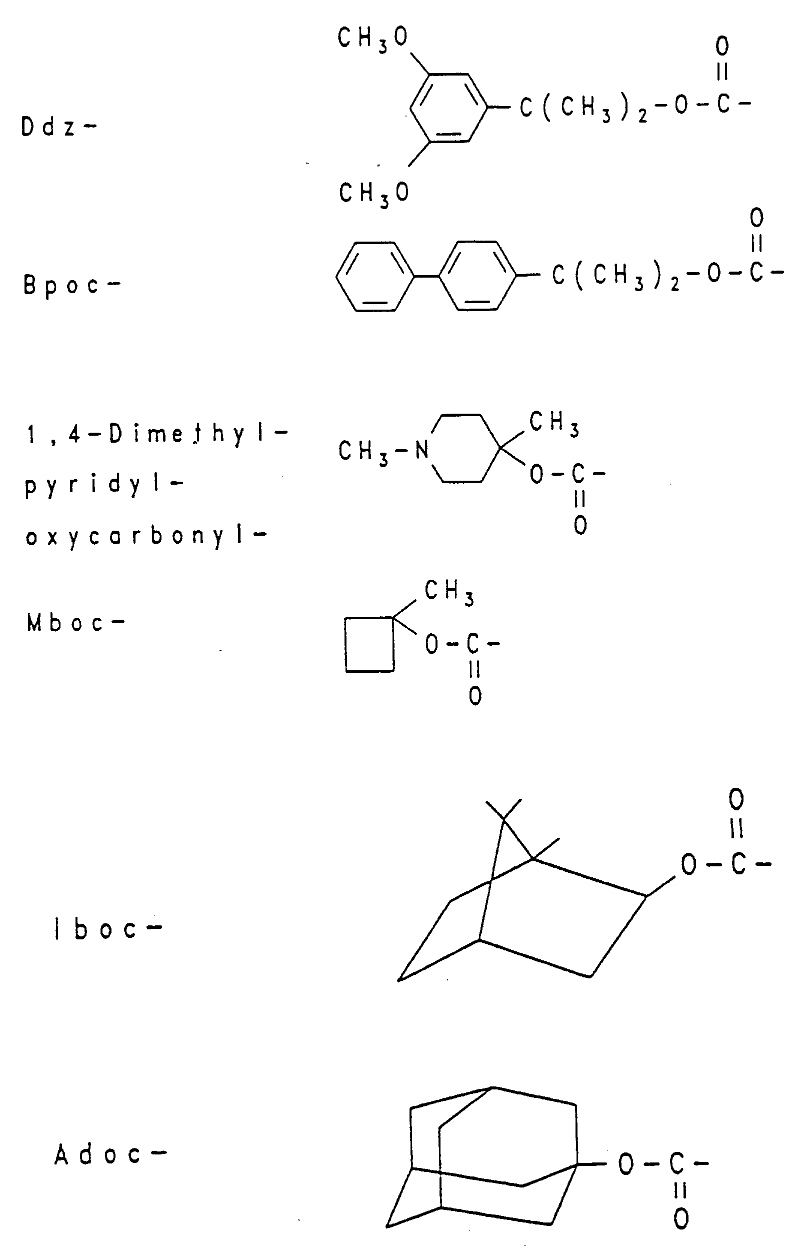 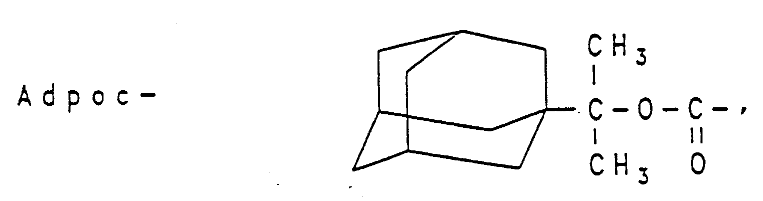 and
R¹ denotes (C₁-C₆)-alkyl, (C₃-C₇)-cycloalkyl, (C₇-C₁₁)-aralkyl or another carboxyl-protecting group, which comprises subjecting a compound of the formula II 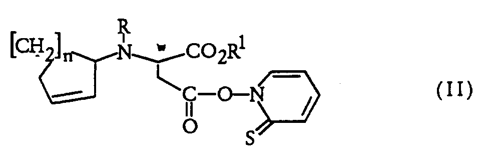 in which n, R and R¹ are as defined above to free-radical decarboxylation by heating to 40 to 190°C in a suitable solvent or else without solvent, in the presence or absence of a free-radical initiator.

2. The process as claimed in claim 1, in which compounds of the formula (I) are prepared in which the hydrogen atoms on the bridgehead carbon atoms 3a and (5+n)a have a cis configuration.

3. The process as claimed in claim 1 or 2, in which compounds of the formula (III) are prepared in which the carbon atom in position 2 has the S configuration.

4. The use of a compound of the formula I as in one of claims 1 to 3 in a process for the preparation of a compound of the formula IV 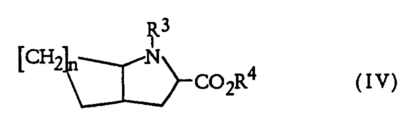 in which
R³ denotes hydrogen or is as defined for R in claim 1, and
R⁴ denotes hydrogen or is as defined for R¹ in claim 1, through treatment of a compound of the formula I with Raney nickel in a suitable solvent, 1 or 2 of the radicals R³ and/or R⁴ which do not denote hydrogen subsequently being replaced, if appropriate, by hydrogen with the aid of acids and/or bases and/or hydrogenolytically, and a compound of the formula IV being converted, if desired, into an ester of the formula IV in which R⁴ is as defined for R in claim 1 by esterification or transesterification.

## Revendications (Revendications pour l'(les) Etat(s) contractant(s) suivant(s): AT, BE, CH, DE, FR, GB, IT, LI, LU, NL, SE)

1. Composé de formule (I) 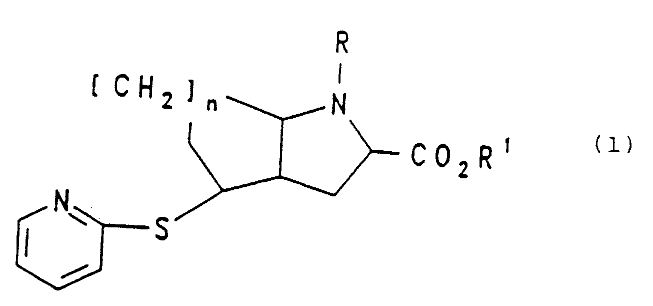 dans laquelle
n = 1, 2 ou 3,
R représente un groupe alcanoyle en C₁-C₆, aryl(C₆-C₁₀)-alcanoyle(C₁-C₄), aroyle en C₆-C₁₀, alcoxy(C₁-C₆)-carbonyle ou aralkyloxy(C₇-C₁₁)-carbonyle, ou un groupe protecteur du type uréthanne choisi parmi 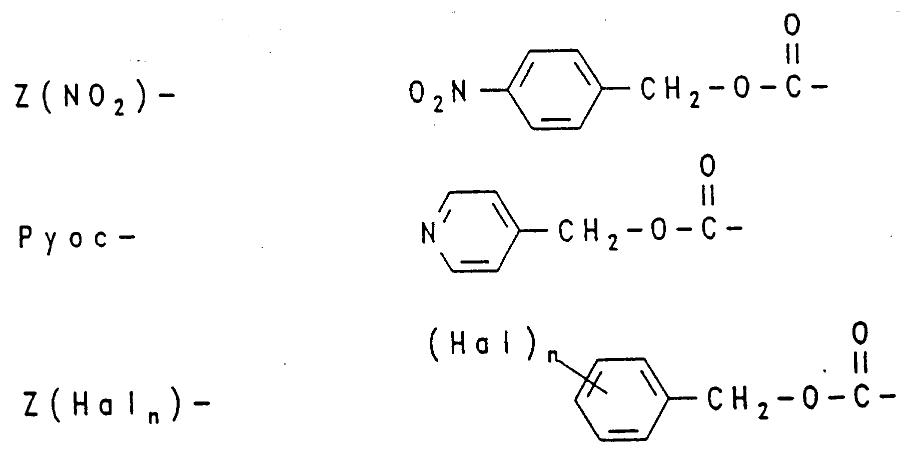 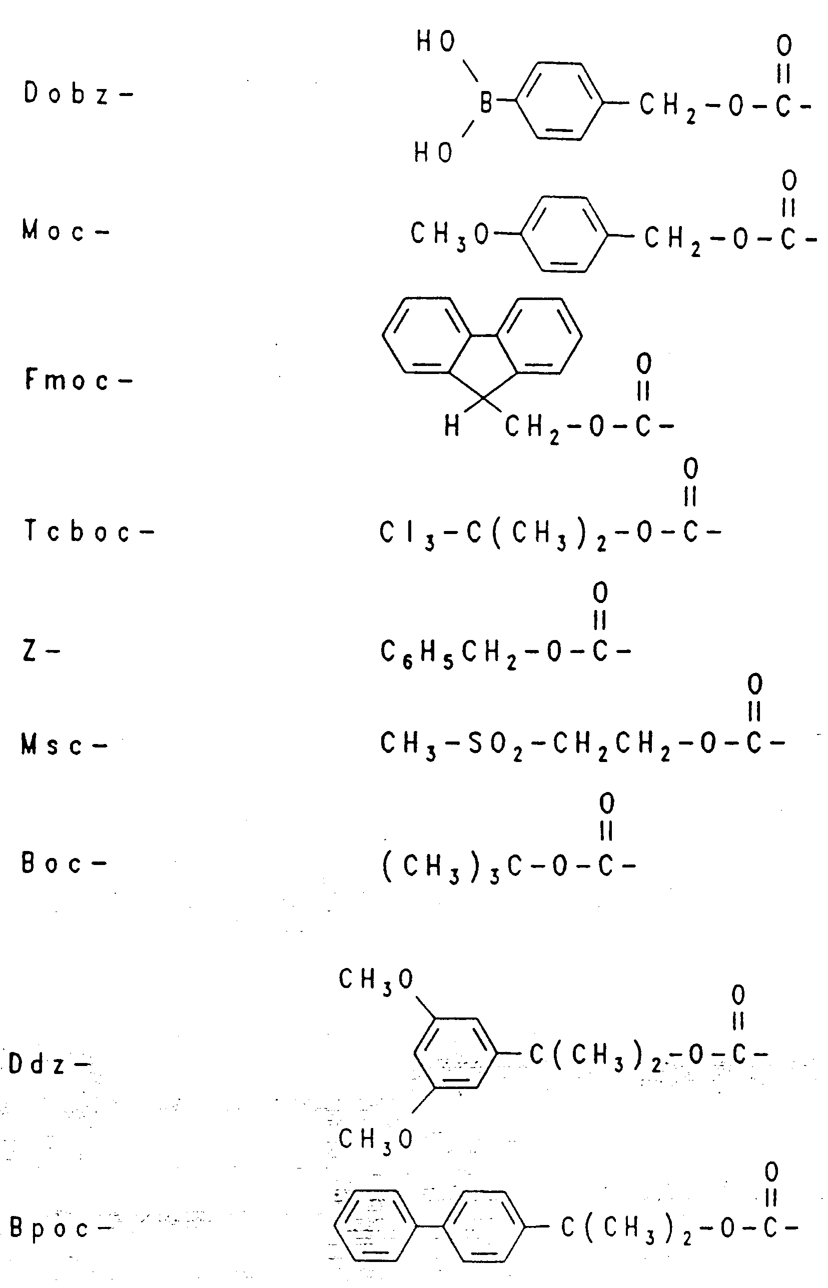 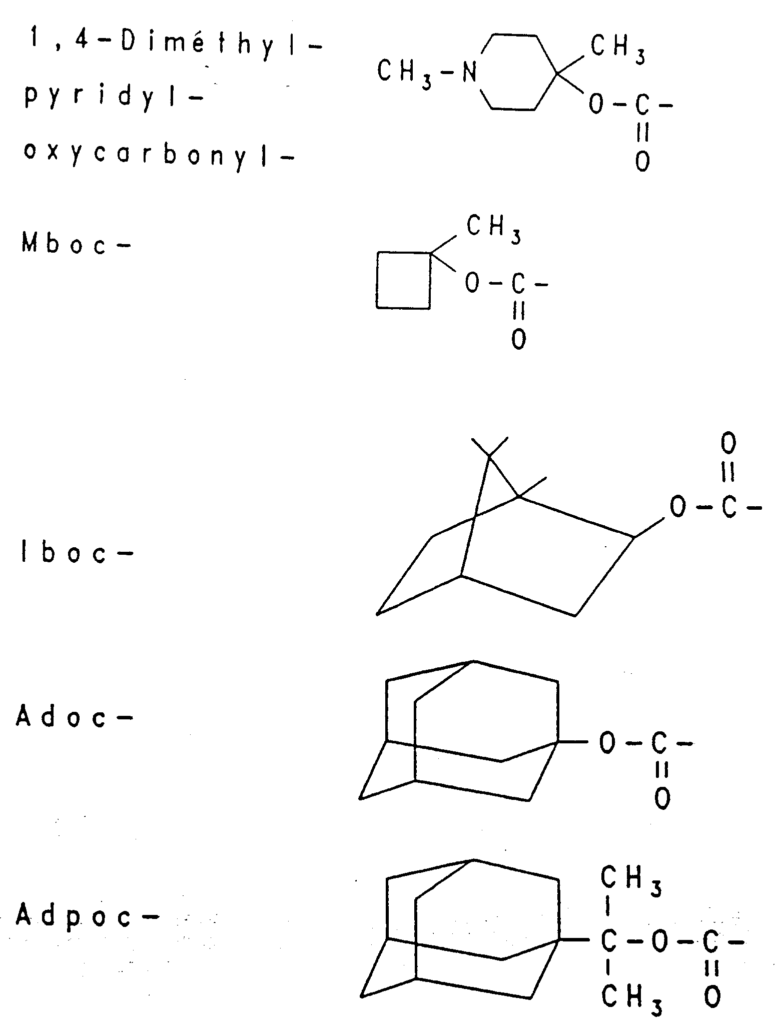
R¹ représente un groupe alkyle en C₁-C₆, cycloalkyle en C₃-C₇, aralkyle en C₇-C₁₁ ou un autre groupe protecteur de fonction carboxy.

2. Composé de formule I selon la revendication 1, dans lequel les atomes d'hydrogène sur les atomes de carbone de tête de pont 3a et (5+n)a sont en configuration cis.

3. Composé de formule I selon la revendication 1 ou 2, dans lequel l'atome de carbone en position 2 présente la configuration S.

4. Procédé pour la préparation d'un composé de formule I selon la revendication 1, caractérisé en ce que l'on effectue une décarboxylation radicalaire d'un composé de formule II 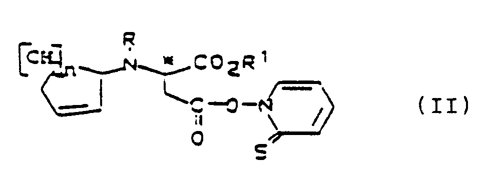 dans laquelle n, R et R¹ sont tels que définis dans la revendication 1,
par chauffage à 40-190°C, dans un solvant convenable ou bien sans solvant, éventuellement en présence d'un initiateur de radicaux libres.

5. Composé de formule III 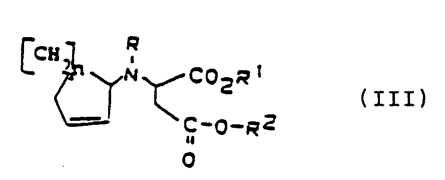 dans laquelle n, R et R¹ sont tels que définis dans la revendication 1, et
R² représente le groupe 2-thioxo-2H-[1]pyridyle ou un groupe alcoxy(C₁-C₆)-carbonyle.

6. Utilisation d'un composé de formule 1 selon une ou plusieurs des revendications 1 à 3, dans un procédé pour la préparation d'un composé de formule IV 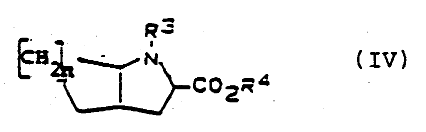 dans laquelle
R³ représente un atome d'hydrogène ou est défini comme R dans la revendication 1, et
R⁴ représente un atome d'hydrogène ou est défini comme R¹ dans la revendication 1,
par traitement d'un composé de formule I par du nickel de Raney dans un solvant approprié, en remplaçant ensuite éventuellement par un(des) atome(s) d'hydrogène 1 ou 2 des radicaux R³ et/ou R⁴, qui ne représentent pas des atomes d'hydrogène, à l'aide d'acides et/ou de bases et/ou par hydrogénolyse, et éventuellement en convertissant par estérification ou transestérification un composé de formule IV en un ester de formule IV dans lequel R⁴ est défini comme R dans la revendication 1.

## Revendications (Revendications pour l'(les) Etat(s) contractant(s) suivant(s): ES, GR)

1. Procédé pour la préparation d'un composé de formule (I) 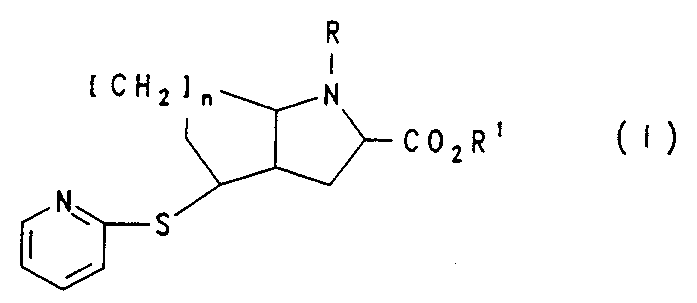 dans laquelle
n = 1 2 ou 3,
R représente un groupe alcanoyle en C₁-C₆, aryl(C₆-C₁₀)-alcanoyle(C₁-C₄), aroyle en C₆-C₁₀, alcoxy(C₁-C₆)-carbonyle ou aralkyloxy(C₇-C₁₁)-carbonyle, ou un groupe protecteur du type uréthanne choisi parmi 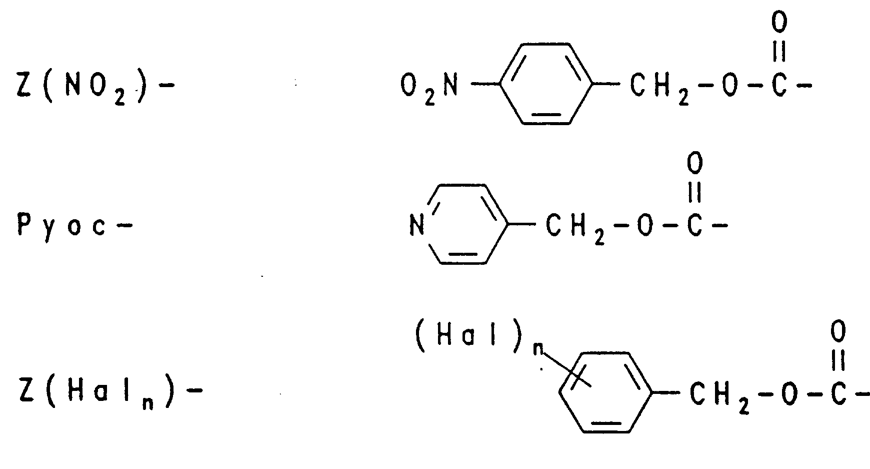 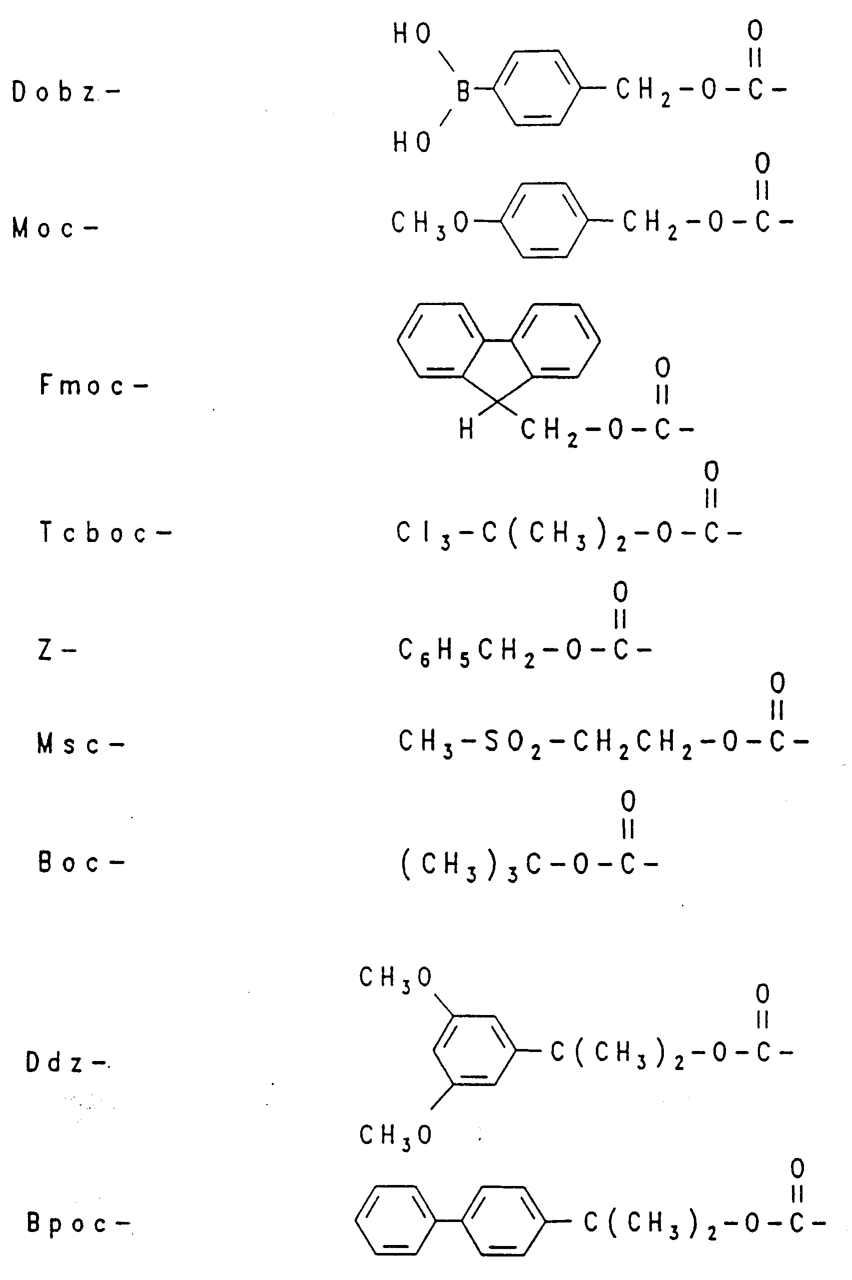 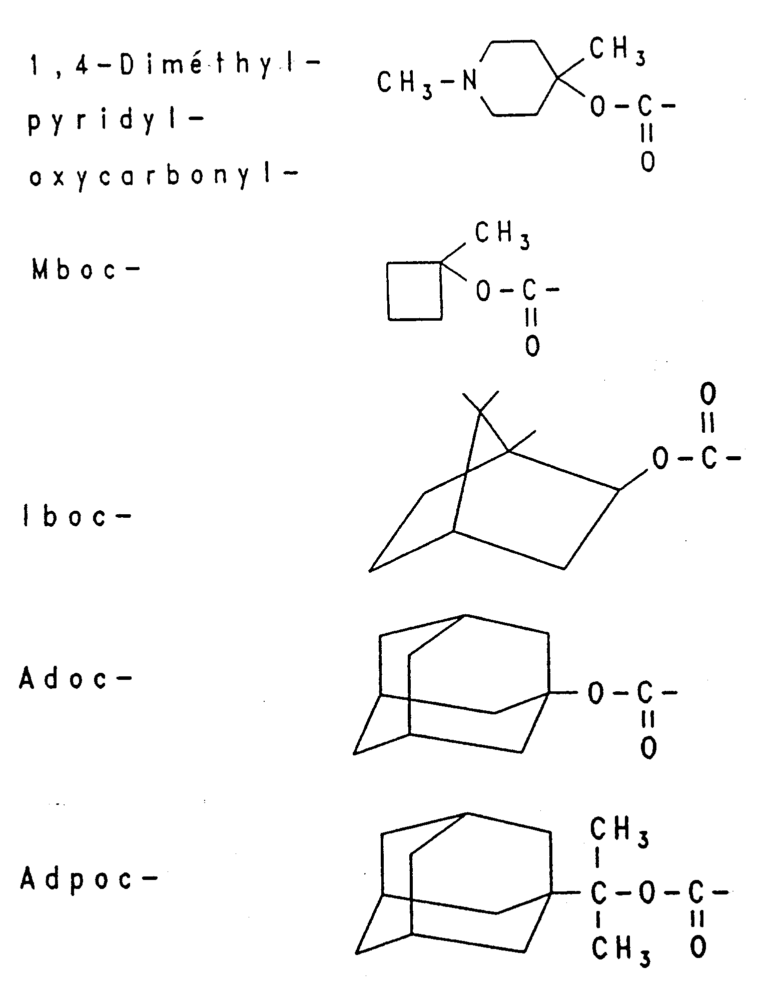
R¹ représente un groupe alkyle en C₁-C₆, cycloalkyle en C₃-C₇, aralkyle en C₇-C₁₁ ou un autre groupe protecteur de fonction carboxy,
caractérisé en ce que l'on effectue une décarboxylation radicalaire d'un composé de formule II 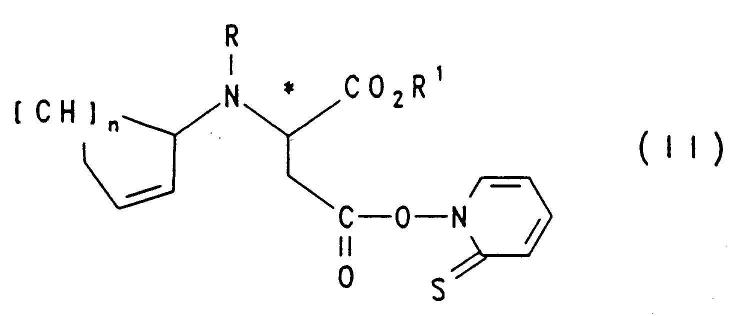 dans laquelle n, R et R¹ sont tels que définis plus haut,
par chauffage à 40-190°C, dans un solvant convenable ou bien sans solvant, éventuellement en présence d'un initiateur de radicaux libres.

2. Procédé selon la revendication 1, dans lequel on prépare des coomposés de formule (I) dans lesquels les atomes d'hydrogène sur les atomes de carbone de tête de pont 3a et (5+n)a sont en configuration cis.

3. Procédé selon la revendication 1 ou 2, dans lequel on prépare des composés de formule (III) dans lequels l'atome de carbone en position 2 présente la configuration S.

4. Utilisation d'un composé de formule 1 selon l'une des revendications 1 à 3, dans un procédé pour la préparation d'un composé de formule IV 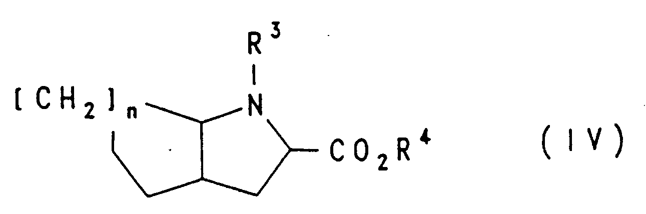 dans laquelle
R³ représente un atome d'hydrogène ou est défini comme R dans la revendication 1, et
R⁴ représente un atome d'hydrogène ou est défini comme R¹ dans la revendication 1,
par traitement d'un composé de formule I par du nickel de Raney dans un solvant approprié, en remplaçant ensuite éventuellement par un(des) atome(s) d'hydrogène 1 ou 2 des radicaux R³ et/ou R⁴, qui ne représentent pas des atomes d'hydrogène, à l'aide d'acides et/ou de bases et/ou par hydrogénolyse, et éventuellement en convertissant par estérification ou transestérification un composé de formule IV en un ester de formule IV dans lequel R⁴ est défini comme R dans la revendication 1.
